# EUROPEAN PATENT SPECIFICATION

(11) **EP 1 203 014 B1**
(45) Date of publication and mention of the grant of the patent: **13.10.2004**
(21) Application number: 00952495.0
(22) Date of filing: 04.08.2000
(51) Int. Cl.: C07K 7/08, A61K 38/10, G01N 33/68, A61P 7/02

(54) **PEPTIDE ANTAGONISTS OF FACTOR VIIA**
PEPTIDANTAGONISTEN DES FAKTORS VIIA
ANTAGONISTE PEPTIDE DU FACTEUR FVIIa

(30) Priority: 06.08.1999 US 147627 P; 23.08.1999 US 150315 P
(43) Date of publication of application: 08.05.2002
(73) Proprietor: Genentech, Inc., South San Francisco, CA 94080-4990 (US)
(72) Inventor: DENNIS, Mark, S., San Carlos, CA 94070 (US)
(74) Representative: Kiddle, Simon John
(86) International application number: PCT/US2000/021296
(87) International publication number: WO 2001/010892

(56) References cited:
- WO-A-00/04148
- WO-A-97/20939
- US-A- 5 834 244
- DENNIS ET AL: "Peptide exosite inhibitors of factor VIIa as anticoagulants" NATURE,MACMILLAN JOURNALS LTD. LONDON,GB, vol. 404, no. 6777, 30 March 2000 (2000-03-30), pages 465-470, XP002155500 ISSN: 0028-0836

## Description

### Field of the Invention

This invention relates to novel compounds which prevent or block a FVIIa mediated or associated process or event such as the catalytic conversion of FX to FXa, FVII to FVIIa or FIX to FIXa. In particular aspects, the compounds of the invention bind Factor VIIa (FVIIa), its zymogen Factor VII (FVII) and/or block the association of FVII or FVIIa with a peptide compound of the present invention. The invention also relates to pharmaceutical compositions comprising the novel compounds as well as their use in research, diagnostic, therapeutic, and prophylactic methods.

### Description of Related Disclosures

Factor VIIa (FVIIa) is a trypsin-like plasma serine protease that participates in hemostasis through the extrinsic pathway of the coagulation cascade (Davie *et al*., *Biochem.* 30(43):10363-10370 (1991)). FVIIa is converted from its zymogen factor VII (FVII) by proteolysis of a single internal peptide bond. Circulating FVII is a globular protein with an N-terminal γ-carboxyglutamic acid (Gla)-domain, two epidermal growth factor (EGF) domains, and a C-terminal protease domain. Prior to conversion to FVIIa, FVII associates with tissue factor (TF) constitutively expressed on cells separated from plasma by the vascular endothelium (Carson, S. D., and Brozna, J. P., *Blood Coag. Fibrinol*. 4:281-292 (1993)). TF and FVII form a one-to-one protein complex (TF-FVIIa) in the presence of calcium ions (Wildgoose *et al., Biochem.* 32:114-119 (1993)). This association facilitates the proteolysis of FVII to FVIIa at a site (Arg152-Ile153 for human FVII (hFVII)) located between the C-terminal EGF domain (EGF2) and the protease domain (Hagen *et al*., *Proc. Natl*. *Acad. Sci USA*, 83:2412-2416 (1986)). While a number of serine proteases activate FVII *in vitro,* the protease responsible for *in vivo* activation of FVII is not known (Wildgoose *et al*., *supra*).

TF functions as a cofactor for FVIIa with the FVIIa Gla domain interacting at the C-terminal end of TF near the membrane and the FVIIa protease domain situated over the N-terminal domain (Higashi *et al*., *J. Biol. Chem*. 269:18891-18898 (1994)). The structures of the human TF (hTF) extracellular domain and its complex with active site inhibited FVIIa have recently been determined by x-ray crystallography (Harlos *et al*., *Nature* 370:662-666 (1994); Muller *et al*., *Biochemistry* 33:10864 (1994); Banner *et al*., *Nature* 380:41-46 (1996)).

The TF-FVIIa complex constitutes the primary initiator of the extrinsic pathway of blood coagulation (Carson, S. D., and Brozna, J. P., *Blood Coag*. *Fibrinol*. 4:281-292 (1993); Davie, E. W., *et al*., *Biochemistry* 30:10363-10370 (1991); Rapaport, S. I., and Rao, L. V. M., *Arterioscler. Thromb*. 12:1111-1121 (1992)). The complex initiates the extrinsic pathway by activation of FX to Factor Xa (FXa), FIX to Factor IXa (FIXa), and additional FVII to FVIIa. The action of TF-FVIIa leads ultimately to the conversion of prothrombin to thrombin, which carries out many biological functions (Badimon, L., *et al*., *Trends Cardiovasc. Med*. 1:261-267 (1991)). Among the most important functions of thrombin is the conversion of fibrinogen to fibrin, which polymerizes to form a clot. The TF-FVIIa complex also participates as a secondary factor in extending the physiological effects of the contact activation system.

The involvement of these plasma protease systems have been suggested to play a significant role in a variety of clinical manifestations including arterial and venous thrombosis, septic shock, adult respiratory distress syndrome (ARDS), disseminated intravascular coagulation (DIC) and various other disease states (Haskel, E. J., *et al*., *Circulation* 84:821-827 (1991)); Holst, J., *et al*., *Haemostasis* 23(suppl. 1):112-117 (1993); Creasey, A. A., *et al*., *J. Clin. Invest*. 91:2850-2860 (1993); see also, Colman, R. W., *N. Engl. J. Med.* 320:1207-1209 (1989); Bone, R. C., *Arch. Intern. Med*. 152:1381-1389 (1992)).

Antibodies reactive with the protease domain of FVII have been shown to inhibit TF-FVIIa proteolytic function (Dickinson *et al*., *J. Mol. Biol*. 277:959-971 (1998)). Peptides corresponding to the EGF2 domain of factor VII are potent inhibitors of TF-FVIIa mediated activation of FX (Husbyn *et al*., *J. Peptide Res*. 50:475-482 (1997)). Several peptides corresponding to various regions of FVII (for example, amino acid sequence residues 372-337 and 103-112 of hFVII) have been proposed as therapeutic anticoagulants based upon their ability to inhibit TF-FVIIa mediated coagulation (International Publication No. WO 90/03390; International Publication No. WO95/00541). Active site modified FVII variants capable of binding TF have been proposed as pharmaceutical compositions for the prevention of TF/FVIIa mediated coagulation (International Publication No. WO 91/11514). International Publication No. WO 96/40779 describes peptide fragments of TF that inhibit FX activation. U.S. Patent Nos. 5,759,954, 5,863,893, 5,880,256 and 5,834,244 describe variant Kunitz-type serine protease inhibitors that inhibit TF-FVIIa activity and have been shown to prolong tissue factor initiated prothrombin time (PT). This is consistant with the ability of these TF-FVIIa active site inhibitors to prevent FX activation through inhibition of the TF-FVIIa complex.

### Summary of the Invention

The present invention provides compounds and compositions which inhibit a FVII/FVIIa mediated or associated process such as the catalytic conversion of FVII to FVIIa, FIX to FIXa, or FX to FXa and thereby block initial events of the extrinsic pathway of blood coagulation. In addition, the compositions of the present invention are capable of neutralizing the thrombotic effects of endogenous TF by binding to FVII or FVIIa and preventing the TF-FVIIa mediated activation of FX. The compositions of the present invention are therefore useful in therapeutic and prophylactic methods for inhibiting TF-FVIIa mediated or associated processes. Advantageously, the compositions allow for a potent inhibition of FVIIa and the TF-FVIIa complex providing, in preferred embodiments, for low dose pharmaceutical formulations.

Accordingly, the invention provides compounds which, by virtue of binding FVII or FVIIa, inhibit a FVII or FVIIa mediated coagulation event. Such compounds preferably bind FVII or FVIIa with a Kd less than about 100 µM, preferably less than about 100 nM, and preferably do not substantially inhibit the activity of other proteases of the coagulation cascade. The compounds of the present invention can be, for example, peptides or peptide derivatives such as peptide mimetics. Specific examples of such compounds include linear or cyclic peptides and combinations thereof, preferably between about 10 and 100 amino acid residues in length, optionally modified at the N-terminus or C-terminus or both, as well as their salts and derivatives, functional analogues thereof and extended peptide chains carrying amino acids or polypeptides at the termini of the sequences for use in the inhibition of FVIIa mediated activation of FX.

The invention further provides a method for identifying a compound which blocks FVII/FVIIa mediated activation of FX comprising the steps of:
(1) contacting FVII/FVIIa with a peptide compound of the invention in the presence and absence of a candidate compound under conditions which allow specific binding of the peptide compound of the invention to FVII/FVIIa to occur;
(2) detecting the amount of specific binding of the peptide compound of the invention to FVII/FVIIa that occurs in the presence and absence of the candidate compound wherein a decrease in the amount of binding of the peptide compound in the presence of the candidate compound relative to the amount of binding in the absence of the candidate compound is indicative of the ability of the candidate compound to block FVII/FVIIa mediated activation of FX.

The invention further provides a method of inhibiting the activation of FX to FXa comprising contacting FVII with TF under conditions which allow formation of a TF-FVIIa complex in the presence of a peptide compound of the invention (or, according to certain aspects, a compound that prevents the interaction of FVII/FVIIa with a peptide compound of the invention) and further contacting the TF-FVIIa complex with FX. According to this aspect of the invention, the contacting steps occur *in vitro.*

In particular aspects, the invention is directed to combinations of peptide compounds with other peptide compounds or with other proteins, especially serum proteins or peptides. The combinations are prepared with various objectives in mind, including; increasing the affinity or avidity of the peptide compound for FVII/FVIIa, as for example, by the use of various multimerization domains as described herein; increasing the stability of the peptide compound or facilitating its recovery and purification, as for example, by expressing the peptide compound as a Z protein fusion; and improving the therapeutic efficacy of the peptide compound in aspects of the invention involving *in vivo* use of the peptide compound, by for example, increasing or decreasing the serum half life, by for example, fusing the peptide compound to a plasma protein such as serum albumin, an immunoglobulin, apolipoproteins or transferrin (such fusion being made conveniently in recombinant host cells or by the use of bifunctional crosslinking agents).

The invention includes compositions, including pharmaceutical compositions, comprising compounds such as peptides for the treatment of a FVII/FVIIa mediated disorder as well as kits and articles of manufacture. Kits and articles of manufacture preferably include:
(a) a container;
(b) a label on or associated with said container; and
(c) a composition comprising a compound of the present invention contained within said container; wherein the composition is effective for treating a FVII/FVIIa mediated disorder. Preferably, the label on said container indicates that the composition can be used for treating a FVII/FVIIa mediated disorder and the compound in said composition comprises a compound which binds FVII/FVIIa and prevents FVII/FVIIa mediated activation of FX. The kits optionally include accessory components such as a second container comprising a pharmaceutically-acceptable buffer and instructions for using the composition to treat a disorder.

Also disclosed are methods useful in preparation of a medicament for the treatment of coagulopathic disorders, especially those characterized by the involvement of EVII/FVIIa or the TF-FVIIa complex. Therefore, the invention provides use of the compounds of the present invention for preparation of a medicament for treating a FVII/FVIIa or TF-FVIIa mediated disease or disorder in a host in need thereof comprising administering to the host a therapeutically effective amount of a compound of the invention. The methods are useful in preventing, blocking or inhibiting a FVII/FVIIa or TF-FVIIa associated event. In preferred embodiments, the methods of the present invention are employed to prepare a medicament to reduce or prevent the severity of or the degree of tissue injury associated with blood coagulation.

The present invention further provides various dosage forms of the compounds of the present invention, including but not limited to, those suitable for parenteral, oral, rectal and pulmonary administration of a compound. In preferred aspects of the present invention a therapeutic dosage form is provided suitable for inhalation and the invention provides for the therapeutic treatment of diseases or disorders involving a FVII/FVIIa mediated or associated process or event, such as the activation of FX, via pulmonary administration of a compound of the invention. More particularly, the invention is directed to pulmonary administration of the compounds of the invention, especially the peptide compounds, by inhalation. Thus, the present invention provides an aerosol formulation comprising an amount of a compound of the invention, more particularly a peptide compound of the invention, effective to block or prevent a FVII/FVIIa mediated or associated process or event and a dispersant. In one embodiment, the compound of the invention, particularly the peptide compound of the invention, can be provided in a liquid aerosol formulation. Alternatively, the compound can be provided as a dry powder aerosol formulation. Therefore, according to the present invention, formulations are provided which provide an effective noninvasive alternative to other parenteral routes of administration of the compounds of the present invention for the treatment of FVII/FVIIa or TF-FVIIa mediated or associated events.

### Brief Description of the Drawings

Figure 1 shows the inhibition of FX activation by selected peptides:
Figures 2A and 2B. Figure 2A shows the inhibition by various peptides of the TF dependent extrinsic clotting pathway as measured by the dose dependent prolongation of the prothrombin time (PT) in normal human plasma. Figure 2B shows the results (no evidence for prolonging the clotting time) in the surface dependent intrinsic pathway as determined by the activated partial thromboplastin time (APTT) in normal human plasma.
Figure 3 shows the inhibition of TF183b (SEQ ID NO:23) binding to FVIIa or TF-FVIIa by selected peptides.

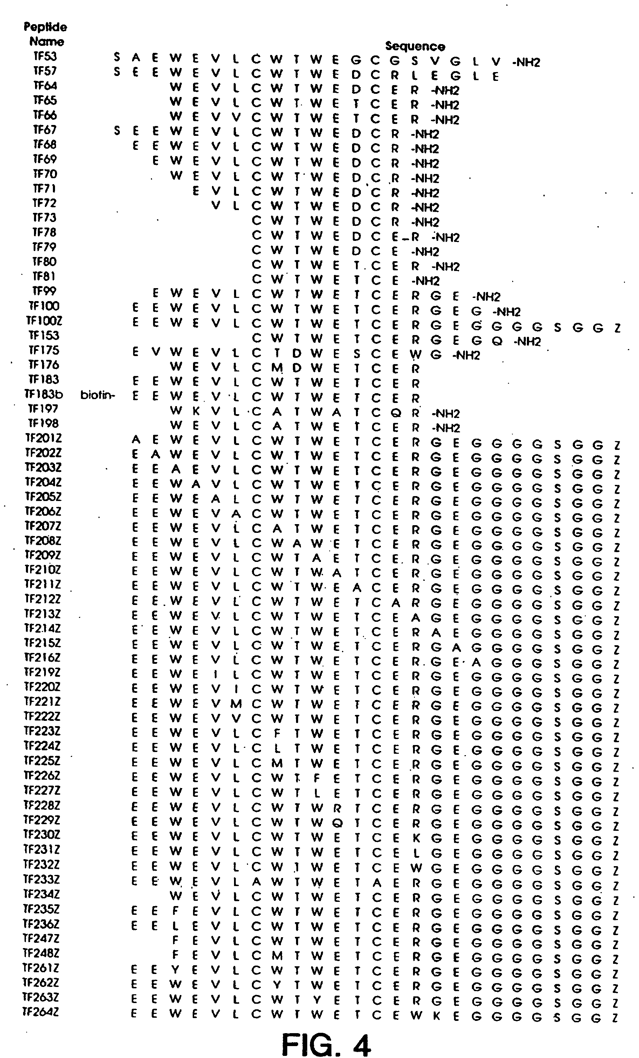

Figure 4 shows the amino acid sequences of selected peptides and their IC₅₀ values for inhibiting FX activation as well as the IC₅₀ values for the inhibition of the binding of TF183b to either FVIIa or TF-FVIIa. "Ac-" denotes CH₃CO-modified N-terminus; "-NH₂" or "-amide" denotes NH₂ modified C-terminus; "aca" denotes aminocaproic acid; "biotin", "bi" or "b" denotes biotin; "Z" denotes the Z consensus domain of protein A (AQHDEAVDNKFNKEQQNAFYEILHLPNLNEEQRNAFIQSLKDDPSQSANLLAEAICKLNDAQAPNVDMN, SEQ ID NO:98). TF53 (SEQ ID NO:1); TF57 (SEQ ID NO:2); TF64 (SEQ ID NO:3); TF65 (SEQ ID NO:4); TF66 (SEQ ID NO:5); TF67 (SEQ ID NO:6); TF68 (SEQ ID NO:7); TF69 (SEQ ID NO:8); TF70 (SEQ ID NO:9); TF71 (SEQ ID NO:10); TF72 (SEQ ID NO:11); TF73 (SEQ ID NO:12); TF78 (SEQ ID NO:13); TF79 (SEQ ID NO:14); TF80 (SEQ ID NO:15); TF81 (SEQ ID NO:16); TF99 (SEQ ID NO:17); TF100 (SEQ ID NO:18); TF100Z (SEQ ID NO:19); TF153 (SEQ ID NO:20); TF175 (SEQ ID NO:21); TF176 (SEQ ID NO:22); TF183 and TF183b (SEQ ID NO:23); TF197 (SEQ ID NO:24); TF198 (SEQ ID NO:25); TF201Z (SEQ ID NO:26); TF202Z (SEQ ID NO:27); TF203Z (SEQ ID NO:28); TF204Z (SEQ ID NO:29); TF205Z (SEQ ID NO:30); TF206Z (SEQ ID NO:31); TF207Z (SEQ ID NO:32); TF208Z (SEQ ID NO:33); TF209Z (SEQ ID NO:34); TF210Z (SEQ ID NO:35); TF211Z (SEQ ID NO:36); F212Z (SEQ ID NO:37); TF213Z (SEQ ID NO:38); TF214Z (SEQ ID NO:39); TF215Z (SEQ ID NO:40); TF216Z (SEQ ID NO:41); TF219Z (SEQ ID NO:42); TF220Z (SEQ ID NO:43); TF221Z (SEQ ID NO:44); TF222Z (SEQ ID NO:45); TF223Z (SEQ ID NO:46); TF224Z (SEQ ID NO:47); TF225Z (SEQ ID NO:48); TF226Z (SEQ ID NO:49); TF227Z (SEQ ID NO:50); TF228Z (SEQ ID NO:51); TF229Z (SEQ ID NO:52); TF230Z (SEQ ID NO:53); TF231Z (SEQ ID NO:54); TF232Z (SEQ ID NO:55); TF233Z (SEQ ID NO:56); TF234Z (SEQ ID NO:57); TF235Z (SEQ ID NO:58); TF236Z (SEQ ID NO:59); TF247Z (SEQ ID NO:60); TF248Z (SEQ ID NO:61); TF261Z (SEQ ID NO:62); TF262Z (SEQ ID NO:63); TF263Z (SEQ ID NO:64); TF264Z (SEQ ID NO:65).

### Detailed Description of the Preferred Embodiments

### Definitions

Terms used in the claims and specification are defined as set forth below unless otherwise specified.

Abbreviations used throughout the description include: FIXa for Factor IXa and FIX for zymogen Factor IX; FXa for Factor Xa and FX for zymogen Factor X; FVII for zymogen factor VII; FVIIa for Factor VIIa; TF for tissue factor; TF-FVIIa for the tissue factor-Factor VIIa complex; FVII/FVIIa for FVII and/or FVIIa; sTF or TF₁₋₂₁₉ for soluble tissue factor composed of the extracellular domain amino acid residues 1-219; TF₁₋₂₄₃ for membrane tissue factor composed of the extracellular domain and transmembrane amino acid residues 1-243 (Paborsky *et al*., *J. Biol*. *Chem*. 266:21911-21916 (1991)); PT for prothrombin time; APTT for activated partial thromboplastin time.

The expressions "agent" and "compound" are used within the scope of the present invention interchangeably and are meant to include any molecule or substance which blocks or prevents the interaction between FVII/FVIIa and a peptide compound of the present invention. Such molecules include small organic and bioorganic molecules, *e.g.*, peptide mimetics and peptide analogs, antibodies, immunoadhesins, proteins, peptides, glycoproteins, glycopeptides, glycolipids, polysaccharides, oligosaccharides, nucleic acids, pharmacological agents and their metabolites, and the like.

Preferred compounds of the present invention include peptide analogs or mimetics of the peptide compounds'of the present invention. These include, for example, peptides containing non-naturally occurring amino acids provided the compound retains FVII/FVIIa inhibitory activity as described herein. Similarly, peptide mimetics and analogs may include non-amino acid chemical structures that mimic the structure of the peptide compounds of the present invention and retain the FVII/VIIa inhibitory activity described. Such compounds are characterized generally as exhibiting similar physical characteristics such as size, charge or hydrophobicity that is present in the appropriate spacial orientation as found in the peptide compound counterparts. A specific example of peptide mimetic compound is a compound in which the amide bond between one or more of the amino acids is replaced, for example, by a carbon-carbon bond or other bond as is well known in the art (see, for example Sawyer, in *Peptide Based Drug Design* pp. 378-422 (ACS, Washington, D.C., 1995).

The term "peptide" is used herein to refer to constrained (that is, having some element of structure as, for example, the presence of amino acids which initiate a β turn or β pleated sheet, or for example, cyclized by the presence of disulfide bonded Cys residues) or unconstrained (*e*.*g*., linear) amino acid sequences of less than about 50 amino acid residues, and preferably less than about 40 amino acids residues, including multimers, such as dimers thereof or there between. Of the peptides of less than about 40 amino acid residues, preferred are the peptides of between about 10 and about 30 amino acid residues and especially the peptides of about 20 amino acid residues. However, upon reading the instant disclosure, the skilled artisan will recognize that it is not the length of a particular peptide but its ability to bind FVII/FVIIa and compete with the binding of a peptide compound described herein that distinguishes the peptide. Therefore, amino acid sequences of 5, 6, 7, 8, 9, 10, 11, 12, 13, 14, 15, 16, 17, 18, 19, 20, 21, 22, 23, 24 and 25 amino acid residues, for example, are equally likely to be compounds within the context of the present invention.

The term "amino acid" within the scope of the present invention is used in its broadest sense and is meant to include the naturally occurring L α-amino acids or residues. The commonly used one- and three-letter abbreviations for naturally occurring amino acids are used herein (Lehninger, A.L., *Biochemistry,* 2d ed., pp. 71-92, (Worth Publishers, New York, New York, 1975). The term includes D-amino acids as well as chemically modified amino acids such as amino acid analogs, naturally occurring amino acids that are not usually incorporated into proteins such as norleucine, and chemically synthesized compounds having properties known in the art to be characteristic of an amino acid. For example, analogs or mimetics of phenylalanine or proline, which allow the same conformational restriction of the peptide compounds as natural Phe or Pro are included within the definition of amino acid. Such analogs and mimetics are referred to herein as "functional equivalents" of an amino acid. Other examples of amino acids are listed by Roberts and Vellaccio, *The Peptides: Analysis, Synthesis, Biology,* Gross and Meiehofer, Eds., Vol. 5, p. 341 (Academic Press, Inc., New York, New York, 1983), which is incorporated herein by reference.

The term "conservative" amino acid substitution as used within this invention is meant to refer to amino acid substitutions which substitute functionally equivalent amino acids. Conservative amino acid changes result in silent changes in the amino acid sequence of the resulting peptide. For example, one or more amino acids of a similar polarity act as functional equivalents and result in a silent alteration within the amino acid sequence of the peptide. The largest sets of conservative amino acid substitutions include:
(1) hydrophobic: His, Trp, Tyr, Phe, Met, Leu, Ile, Val, Ala;
(2) neutral hydrophilic: Cys, Ser, Thr;
(3) polar: Ser, Thr, Asn, Gln;
(4) acidic/negatively charged: Asp, Glu;
(5) charged: Asp, Glu, Arg, Lys, His;
(6) positively charged: Arg, Lys, His;
(7) basic: His, Lys, Arg;
(8) residues that influence chain orientation: Gly, Pro; and
(9) aromatic: Trp, Tyr, Phe, His.

In addition, structurally similar amino acids can substitute conservatively for some of the specific amino acids. Groups of structurally similar amino acids include: (Ile, Leu, and Val); (Phe and Tyr); (Lys and Arg); (Gln and Asn); (Asp and Glu); and (Gly and Ala). In this regard, it is understood that amino acids are substituted on the basis of side chain bulk, charge and/or hydrophobicity.

Amino acid residues can be further classified as cyclic or noncyclic, aromatic or non aromatic with respect to their side chain groups these designations being commonplace to the skilled artisan.

| Original Residue | Exemplary Conservative Substitution | Preferred Conservative Substitution |
|---|---|---|
| Ala | Val, Leu, Ile | Val |
| Arg | Lys, Gln, Asn | Lys |
| Asn | Gln, His, Lys, Arg | Gln |
| Asp | Glu | Glu |
| Cys | Ser | Ser |
| Gln | Asn | Asn |
| Glu | Asp | Asp |
| Gly | Pro | Pro |
| His | Asn, Gln, Lys, Arg | Arg |
| Ile | Leu, Val, Met, Ala Phe | Leu |
| Leu | Ile, Val Met, Ala, Phe | Ile |
| Lys | Arg, Gln, Asn | Arg |
| Met | Leu, Phe, Ile | Leu |
| Phe | Leu, Val, Ile, Ala | Leu |
| Pro | Gly | Gly |
| Ser | Thr | Thr |
| Thr | Ser | Ser |
| Trp | Tyr | Tyr |
| Tyr | Trp, Phe, Thr, Ser | Phe |
| Val | Ile, Leu, Met, Phe | Leu |
| | Ala | |

Peptides synthesized by the standard solid-phase synthesis techniques described here, for example, are not limited to amino acids encoded by genes for substitutions involving the amino acids. Commonly encountered amino acids which are not encoded by the genetic code include, for example, those described in International Publication No. WO 90/01940 and described in Table I below, as well as, for example, 2-amino adipic acid (Aad) for Glu and Asp; 2-aminopimelic acid (Apm) for Glu and Asp; 2-aminobutyric (Abu) acid for Met, Leu, and other aliphatic amino acids; 2-aminoheptanoic acid (Ahe) for Met, Leu and other aliphatic amino acids; 2-aminoisobutyric acid (Aib) for Gly; cyclohexylalanine (Cha) for Val, and Leu and Ile; homoarginine (Har) for Arg and Lys; 2,3-diaminopropionic acid (Dpr) for Lys, Arg and His; N-ethylglycine (EtGly) for Gly, Pro, and Ala; N-ethylglycine (EtGly) for Gly, Pro, and Ala; N-ethylasparigine (EtAsn) for Asn, and Gln; Hydroxyllysine (Hyl) for Lys; allohydroxyllysine (AHyl) for Lys; 3-(and 4)hydoxyproline (3Hyp, 4Hyp) for Pro, Ser, and Thr; allo-isoleucine (AIle) for Ile, Leu, and Val; ρ-amidinophenylalanine for Ala; N-methylglycine (MeGly, sarcosine) for Gly, Pro, and Ala; N-methylisoleucine (MeIle) for Ile; Norvaline (Nva) for Met and other aliphatic amino acids; Norleucine (Nle) for Met and other aliphatic amino acids; Ornithine (Orn or Or) for Lys, Arg and His; Citrulline (Cit) and methionine sulfoxide (MSO) for Thr, Asn and Gln; -methylphenylalanine (MePhe), trimethylphenylalanine, halo (F, Cl, Br, and I)phenylalanine, trifluoroylphenylalanine, for Phe.

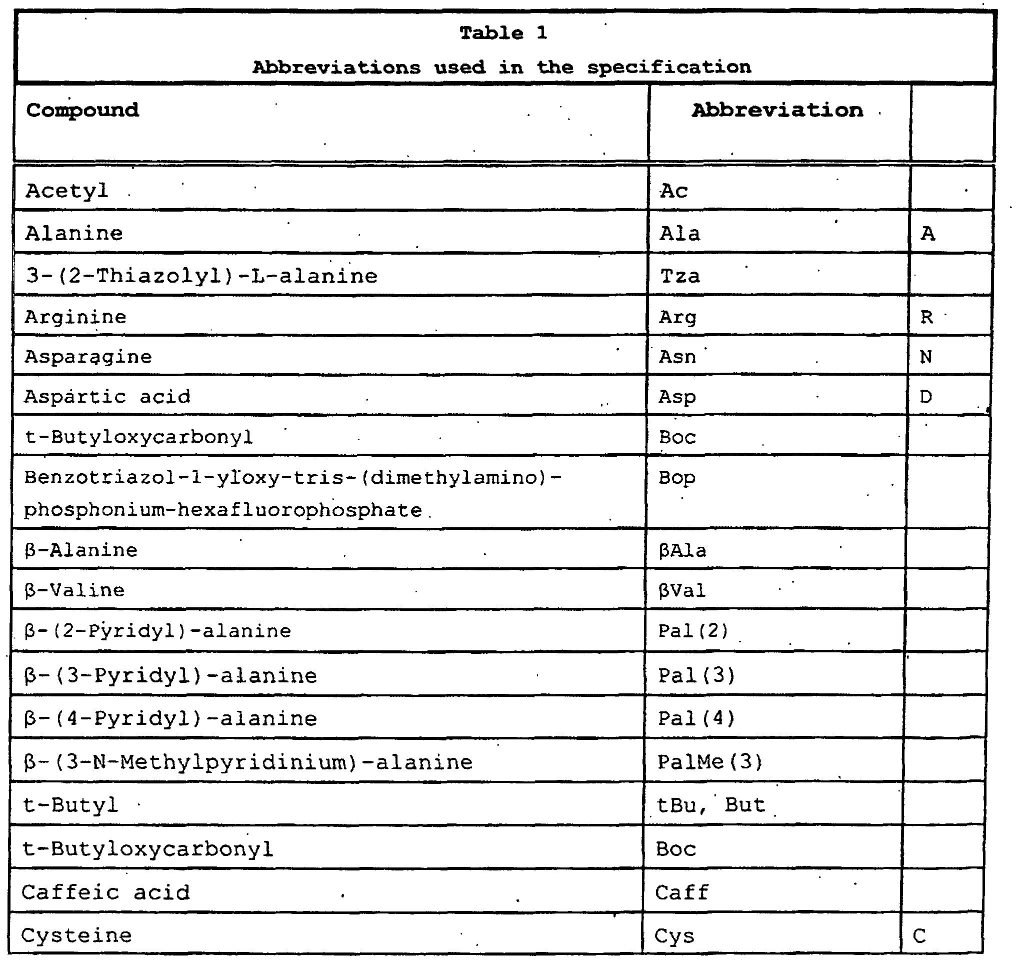

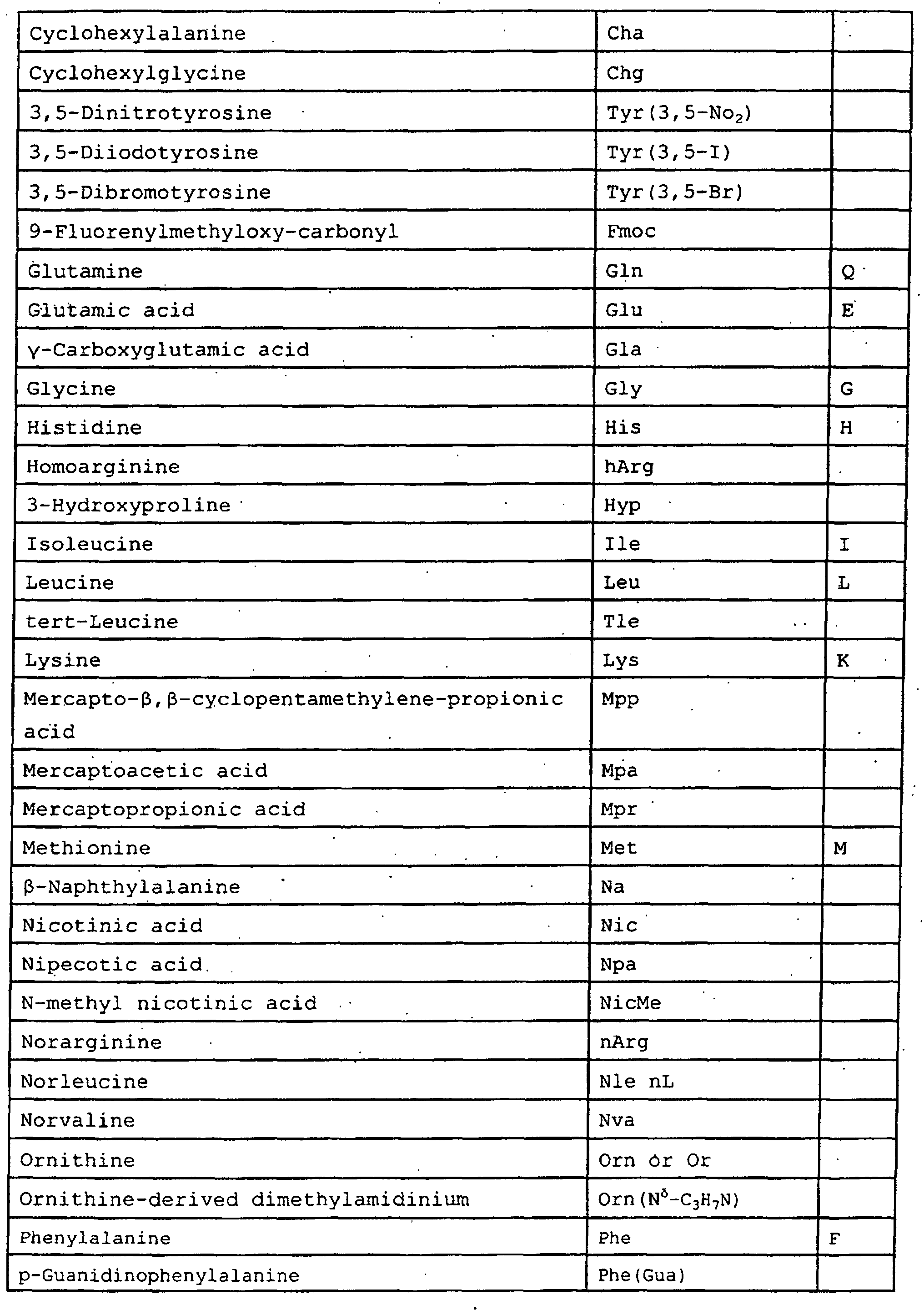

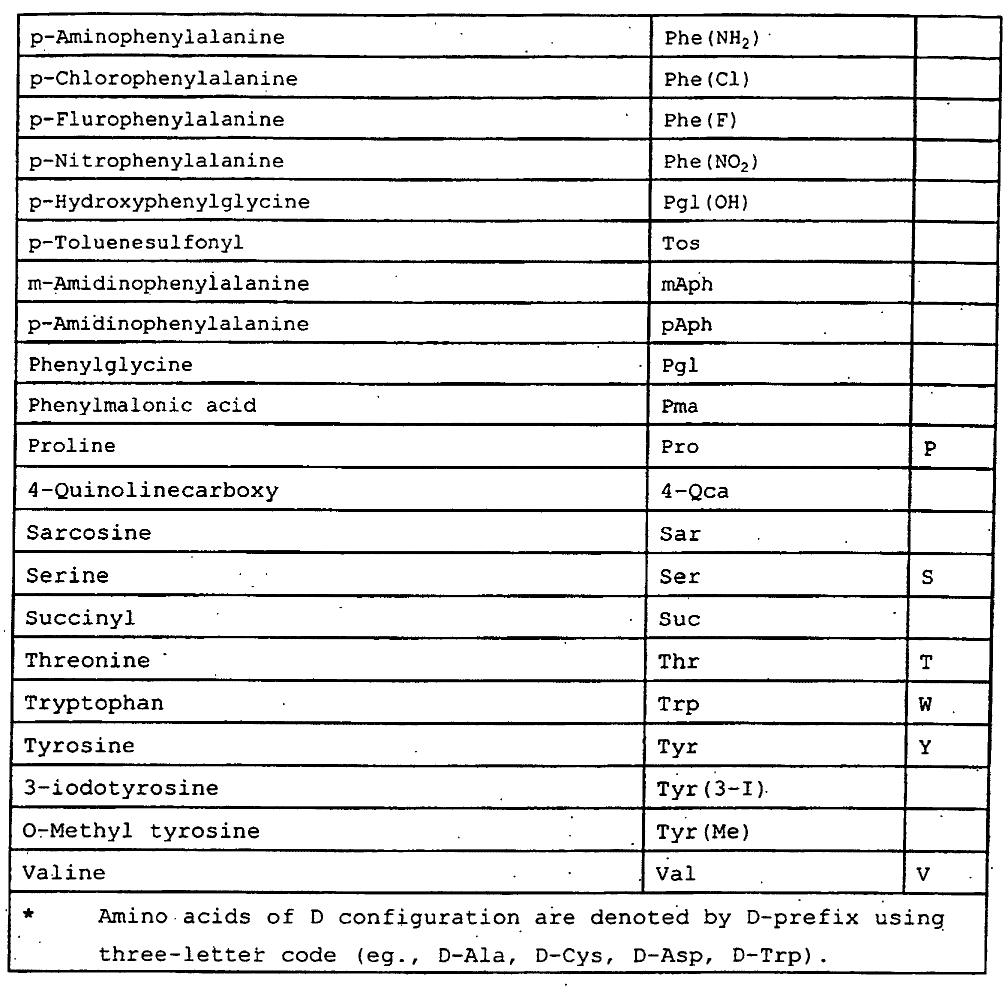

A useful method for identification of certain residues or regions of the compound for amino acid substitution other than those described herein is called alanine scanning mutagenesis as described by Cunningham and Wells, *Science* 244:1081-1085 (1989). Here a residue or group of target residues are identified (*e.g.*, charged residues such as Arg, Asp, His, Lys, and Glu) and replaced by a neutral or negatively charged amino acid to affect the interaction of the amino acids with the surrounding aqueous environment in or outside the cell. Those regions demonstrating functional sensitivity to the substitution are then refined by introducing further or other variations at or for the sites of substitution. Thus while the site for introducing an amino acid sequence variation is predetermined the nature of the mutation per se need not be predetermined. For example, to optimize the performance of a mutation at a given site, Ala scanning or random mutagenesis may be conducted at the target codon or region and the expressed compound screened for the optimal combination of desired activity.

Phage display of protein or peptide libraries offers another methodology for the selection of compounds with improved affinity, altered specificity, or improved stability (Smith, G.P., *Curr. Opin. Biotechnol*. 2:668-673 (1991); Lowman, *Ann. Rev. Biophys. Biomol. Struct.* 26:401-404 (1997)). High affinity proteins, displayed in a monovalent fashion as fusions with the M13 gene III coat protein (Clackson, T., *et al*., *Trends Biotechnol*. 12:173-183 (1994)), can be identified by cloning and sequencing the corresponding DNA packaged in the phagemid particles after a number of rounds of binding selection.

Other compounds include the fusion to the N - or C- terminus of the compounds described herein of immunogenic polypeptides, *e.g*., bacterial polypeptides such as beta lactamase or an enzyme encoded by *E. coli* Trp locus or yeast protein, other polypeptides such as the Z-domain of protein-A, and C-terminal fusion with proteins having a long half-life such as immunoglobulin constant region or other immunoglobulin regions, albumin, or ferritin, as described in WO 89/02922, published 6 April 1989. Further, free functional groups on the side chains of the amino acid residues can also be modified by amidation, acylation or other substitution, which can, for example, change the solubility of the compounds without affecting their activity. "Ac-" for example denotes a CH₃CO- modified N terminus and "-NH2" a -NH₂ modified C-terminus.

Preferred amino acid sequences within the context of the present invention are non-naturally occurring amino acid sequences. By non-naturally occurring is meant that the amino acid sequence is not found in nature. Preferred are non-naturally occurring amino acid sequences of between about 10 and 30 amino acid residues and preferably about 20 amino acid residues. These include peptides, peptide analogs and mimetics containing naturally as well as non-naturally occurring amino acids. Especially preferred are sequences as described above consisting of naturally occurring amino acids.

The term "multimerization domain," as used in particular aspects of the present invention, is meant to refer to the portion of the molecule to which the compound, especially the peptide compound, is joined, either directly or through a "linker domain." The multimerization domain is an amino acid domain which, according to preferred embodiments, facilitates the interaction of two or more multimerization domains. While the multimerization domain promotes the interaction between two or more multimerization domains, there is no requirement within the context of the present invention that the peptide joined to a multimerization domain be present as a portion of a multimer.

According to preferred aspects of the present invention, the multimerization domain is a polypeptide which promotes the stable interaction of two or more multimerization domains. By way of example and not limitation, a multimerization domain may be an immunoglobulin sequence, such as an immunoglobulin constant region, a leucine zipper, a hydrophobic region, a hydrophilic region, a polypeptide comprising a free thiol which forms an intermolecular disulfide bond between two or more multimerization domains or, for example, a "protuberance-into-cavity" domain described in U.S. Patent No. 5,731,168. In that patent, protuberances are constructed by replacing small amino acid side chains from the interface of a first polypeptide with a larger side chain (for example, a tyrosine or tryptophan). Compensatory cavities of identical or similar size to the protuberances are optionally created on the interface of a second polypeptide by replacing large amino acid side chains with smaller ones (for example, alanine or threonine).

Therefore, in a preferred aspect, the multimerization domain provides that portion of the molecule which promotes or allows stable interaction of two or more multimerization domains and promotes or allows the formation of dimers and other multimers from monomeric multimerization domains. Preferably, according to this aspect of the invention, multimerization domains are immunoglobulin constant region domains. Immunoglobulin constant domains provide the advantage of improving *in vivo* circulating half-life of the compounds of the invention and optionally allow the skilled artisan to incorporate an "effector function" as described herein below into certain aspects of the invention.

Throughout the present specification and claims, the numbering of the residues in an immunoglobulin heavy chain is that of the EU index, as in Kabat *et al., Sequences of Proteins of Immunological Interest*, 5th Ed. (Public Health Service, National Institutes of Health, Bethesda, Maryland, 1991), expressly incorporated herein by reference. The "EU index as in Kabat" refers to the residue numbering of the human IgGl EU antibody.

"Antibodies" (Abs) and "immunoglobulins" (Igs) are typically glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

"Antibodies" and "immunoglobulins" are usually heterotetrameric glycoproteins of about 150,000 Daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has an amino (N) terminal variable domain (VH) followed by carboxyl(C) terminal constant domains. Each light chain has a variable N-terminal domain (VL) and a C-terminal constant domain; the constant domain of the light chain (CL) is aligned with the first constant domain (CH1) of the heavy chain, and the light chain variable domain is aligned with the variable domain of the heavy chain. According to the domain definition of immunoglobulin polypeptide chains, light (L) chains have two conformationally similar domains VL and CL; and heavy chains have four domains (VH, CH1, CH2, and CH3) each of which has one intrachain disulfide bridge.

Depending upon the amino acid sequence of the constant (C) domain of the heavy chains, immunoglobulins can be assigned to different classes. There are five major classes of immunoglobulins: IgA, IgD, IgE, IgG, and IgM. The immunoglobulin class can be further divided into subclasses (isotypes), *e.g.*, IgG₁, IgG₂, IgG₃, IgG₄, IgA₁, and IgA₂. The heavy-chain constant domains that correspond to the different classes of immunoglobulins are α, δ, ε, γ, and µ domains respectively. The light chains of antibodies from any vertebrate species can be assigned to one of two distinct types called kappa (κ) or lambda (λ), based upon the amino acid sequence of their constant domains. Sequence studies have shown that the µ chain of IgM contains five domains VH, CHµ1, CHµ2, CHµ3, and CHµ4. The heavy chain of IgE (ε) also contains five domains.

The subunit structures and three-dimensional configurations of different classes of immunoglobulins are well known. Of these IgA and IgM are polymeric and each subunit contains two light and two heavy chains. The heavy chain of IgG (γ) contains a length of polypeptide chain lying between the CHγ1 and CHγ2 domains known as the hinge region. The α chain of IgA has a hinge region containing an O-linked glycosylation site and the µ and ε chains do not have a sequence analogous to the hinge region of the γ and α chains, however, they contain a fourth constant domain lacking in the others. The domain composition of immunoglobulin chains can be summarized as follows:
Light Chain
   λ = Vλ Cλ
   κ = Vκ Cκ
Heavy Chain IgG
   (γ) = VH CHγ1, hinge CHγ2 CHγ3
   IgM (µ) = VH CHµ1 CHµ2 CHµ3 CHµ4
   IgA (α) = VH CHα1 hinge CHα2 CHα3
   IgE (ε) = VH CHε1 CHε2 CHε3 CHε4
   IgD (δ) = VH CHδ1 hinge CHδ2 CHδ3

The "CH2 domain" of a human IgG Fc region (also referred to as "Cγ2" domain) usually extends from about amino acid 231 to about amino acid 340. The CH2 domain is unique in that it is not closely paired with another domain. Rather, two N-linked branched carbohydrate chains are interposed between the two CH2 domains of an intact native IgG molecule.

The "CH3 domain" comprises the stretch of residues C-terminal to a CH2 domain in an Fc region (*i*.*e*., from about amino acid residue 341 to about amino acid residue 447 of an IgG).

"Hinge region" is generally defined as stretching from Glu216 to Pro230 of human IgG1 (Burton, *Molec. Immunol*. 22:161-206 (1985)). Hinge regions of other IgG isotypes may be aligned'with the IgG1 sequence by placing the first and last cysteine residues forming inter-heavy chain S-S bonds in the same positions.

The "lower hinge region" of an Fc region is normally defined as the stretch of residues immediately C-terminal to the hinge region, *i.e.*, residues 233 to 239 of the Fc region.

A TF-FVIIa mediated or associated process or event, or equivalently, an activity associated with plasma FVII/FVIIa, according to the present. invention is any event which requires the presence of FVIIa. The general mechanism of blood clot formation is reviewed by Ganong, in *Review of Medical Physiology,* 13th ed., pp.411-414 (Lange, Los Altos, California, 1987). Coagulation requires the confluence of two processes, the production of thrombin which induces platelet aggregation and the formation of fibrin which renders the platelet plug stable. The process comprises several stages each requiring the presence of discrete proenzymes and procofactors. The process ends in fibrin crosslinking and thrombus formation. Fibrinogen is converted to fibrin by the action of thrombin. Thrombin, in turn, is formed by the proteolytic cleavage of prothrombin. This proteolysis is effected by FXa which binds to the surface of activated platelets and in the presence' of FVa and calcium, cleaves prothrombin. TF-FVIIa is required for the proteolytic activation of FX by the extrinsic pathway of coagulation. Therefore, a process mediated by or associated with TF-FVIIa, or an activity associated with FVII/FVIIa includes any step in the coagulation cascade from the formation of the TF-FVIIa complex to the formation of a fibrin platelet clot and which initially requires the presence FVII/FVIIa. For example, the TF-FVIIa complex initiates the extrinsic pathway by activation of FX to FXa, FIX to FIXa, and additional FVII to FVIIa.

TF-FVIIa mediated or associated process, or FVII/FVIIa mediated or associated activity, can be conveniently measured employing standard assays, such as those described in Roy, S., *J*. *Biol*. *Chem*. 266:4665-4668 (1991), O'Brien, D., *et al*., *J. Clin. Invest*. 82:206-212 (1988), Lee *et al*., *Biochemistry* 36:5607-5611 (1997), Kelly *et al*., *J. Biol. Chem*. 272:17467-17472 (1997), for the conversion of chromogenic substrates or Factor X to Factor Xa in the presence of Factor VII and other necessary reagents.

A TF-FVIIa related disease or disorder is meant to include chronic thromboembolic diseases or disorders associated with fibrin formation including vascular disorders such as deep venous thrombosis, arterial thrombosis, stroke, tumor metastasis, thrombolysis, arteriosclerosis and restenosis following angioplasty, acute and chronic indications such as inflammation, septic shock, septicemia, hypotension, adult respiratory distress syndrome (ARDS), disseminated intravascular coagulapathy (DIC) and other diseases. The TF-FVIIa related disorder is not limited to *in vivo* coagulopathic disorders such as those named above but includes inappropriate or undesirable coagulation related to circulation of blood through stents or artificial valves or related to extracorporeal circulation including blood removed in-line from a patient in such processes as dialysis procedures, blood filtration, or blood bypass during surgery.

As used herein, the term "pulmonary administration" refers to administration of a formulation of the invention through the lungs by inhalation. As used herein, the term "inhalation" refers to intake of air to the alveoli. In specific examples, intake can occur by self-administration of a formulation of the invention while inhaling, or by administration via a respirator, *e.g.*, to a patient on a respirator. The term "inhalation" used with respect to a formulation of the invention is synonymous with "pulmonary administration."

As used herein, the term "parenteral" refers to introduction of a compound of the invention into the body by other than the intestines, and in particular, intravenous (i.v.), intraarterial (i.a.), intraperitoneal (i.p.), intramuscular (i.m.), intraventricular, and subcutaneous (s.c.) routes.

As used herein, the term "aerosol" refers to suspension in the air. In particular, aerosol refers to the particlization of a formulation of the invention and its suspension in the air. According to the present invention, an aerosol formulation is a formulation comprising a compound of the present invention that is suitable for aerosolization, *i.e.*, particlization and suspension in the air, for inhalation or pulmonary administration.

The term "treatment" as used within the context of the present invention is meant to include therapeutic treatment as well as prophylactic, or suppressive measures for the disease or disorder. Thus, for example, the term treatment includes the administration of an agent prior to or following the onset of a disease or disorder thereby preventing or removing all signs of the disease or disorder. As another example, administration of the agent after clinical manifestation of the disease to combat the symptoms of the disease comprises "treatment" of the disease. Further, administration of the agent after onset and after clinical symptoms have developed where administration affects clinical parameters of the disease or disorder, such as the degree of tissue injury or the amount or extent of leukocyte trafficking and perhaps amelioration of the disease, comprises "treatment" of the disease.

Those "in need of treatment" include mammals, such as humans, already having the disease or disorder, including those in which the disease or disorder is to be prevented.

The term "acyl" is used in its broadest sense to mean saturated or unsaturated, linear, branched or cyclic chains of about 1 to about 16 carbon atoms, which contain a carboxyl group. Thus the term acyl includes, for example, groups such as formyl, acetyl, benzoyl and the like. The term "hydrophobic acyl" group refers to a R1-C(=O)- group wherein R1 is an alkyl, aryl or other non-polar group.

### Modes for Carrying out the Invention

### Selection of Compounds

The present invention provides compounds and compositions which inhibit a FVII/FVIIa mediated or associated process such as the catalytic conversion of FVII to FVIIa, FIX to FIXa, or FX to FXa and thereby block initial events of the extrinsic pathway of blood coagulation. Preferred compounds of the present invention are distinguished by their ability compete with a peptide compound of Figure 4 for binding FVII/FVIIa and may 'be selected as follows.

For *in vitro* assay systems to determine whether a compound has the "ability" to compete with a peptide compound as noted above, the skilled artisan can employ any of a number of standard competition assays. Such procedures include but are not limited to competitive assay systems using techniques such as radioimmunoassays, enzyme immunoassays (EIA), preferably the enzyme linked immunosorbent assay (ELISA), "sandwich" immunoassays, immunoradiometric assays, fluorescent immunoassays, and immunoelectrophoresis assays, to name but a few.

For these purposes the selected peptide compound of Figure 8 will be labeled with a detectable moiety (the detectably labeled peptide compound herein called the "tracer") and used in a competition assay with a..candidate compound for binding FVII/FVIIa. Numerous detectable labels are available which can be preferably grouped into the following categories:
(a) Radioisotopes, such as ³⁵S, ¹⁴C, ¹²⁵I, ³H, and ¹³¹I. The peptide compound can be labeled with the radioisotope using the techniques described in *Current Protocols in Immunology,* Volumes 1 and 2, Coligen *et al.,* Ed. (Wiley-Interscience, New York, New York, 1991), for example, and radioactivity can be measured using scintillation counting.
(b) Fluorescent labels such as rare-earth chelates (europium chelates) or fluorescein and its derivatives, rhodamine and its derivatives, dansyl, Lissamine, phycoerythrin and Texas Red are available. 'The fluorescent labels can be conjugated to the peptide compounds using the techniques disclosed in *Current Protocols in Immunology, supra,* for example. Fluorescence can be quantified using a fluorimeter.
(c) Various enzyme-substrate labels are available and U.S. Patent No. 4,275,149 provides a review of some of these. The enzyme preferably catalyses a chemical alteration of the chromogenic substrate which can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (e.g., firefly luciferase and bacterial luciferase; U.S. Patent No. 4,737,456), luciferin, 2,3-dihydrophthalazinediones, malate dehydrogenase; urease, peroxidase such as horseradish peroxidase (HRP), alkaline phosphatase, β-galactosidase, glucoamylase, lysozyme, saccharide oxidases (*e.g.*, glucose oxidase, galactose oxidase, and glucose-6-phosphate dehydrogenase), heterocyclic oxidases (such as uricase and xanthine oxidase), lactoperoxidase, microperoxidase, and the like.

Examples of enzyme-substrate combinations include, for example:
(i) Horseradish peroxidase (HRP) with hydrogen peroxidase as a substrate, wherein the hydrogen peroxidase oxidizes a dye precursor (*e*.*g*., ABTS, orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) β-D-galactosidase (β-D-Gal) with a chromogenic substrate (*e.g.*, p-nitrophenyl-β-D-galactosidase) or fluorogenic substrate 4-methylumbelliferyl-β-D-galactosidase.

According to a particular assay, the tracer is incubated with immobilized FVII/FVIIa in varying concentration of unlabeled candidate compound. Increasing concentrations of successful candidate compound effectively compete with binding of the tracer to immobilized FVII/FVIIa. The concentration of unlabeled candidate compound at which 50% maximal tracer is displaced is referred to as the IC₅₀ and reflects the FVII/FVIIa binding affinity of the candidate compound. Therefore a candidate compound with an IC₅₀ of 1 mM displays a substantially weaker interaction with FVII/FVIIa than a candidate peptide with an IC₅₀ of 1 µM.

Accordingly, the invention provides compound "having the ability to compete" for binding FVII/FVIIa in an *in vitro* assay as described. Preferably the compound has an "IC₅₀" for FVIIa of less than 1 µM. Preferred among these compound are compounds having an IC₅₀ of less than about 100 nM and preferably less than about 10 nM or less than about 1 nM. In further preferred embodiments according to this aspect of the invention, the compounds display an IC₅₀ for FVIIa of less than about 100 pM and more preferably less than about 10 pM.

A preferred *in vitro* assay for the determination of a candidate compound's ability to compete with a peptide compound of Figure 4 is as follows and is described more fully in Example 1. The ability of peptides to compete with tracer for binding to FVIIa is monitored using an ELISA. Dilutions of candidate peptide in buffer are added to microtiter plates coated with TF-FVIIa (as described in the Example Sections) along with tracer for 1 hr. The microtiter plate is washed with wash buffer and the amount of tracer bound to FVIIa measured.

In particular embodiments the tracer is SEQ ID NO: 4 and is added to the FVII/FVIIa coated plated at a concentration of 10 µM. In another preferred embodiment the tracer (SEQ ID NO: 4) is added to the FVII/FVIIa coated plate at a concentration of 5 nM.

Compounds selected in this way are then tested for their ability to inhibit or block FVII/FVIIa activation of FX. The term "inhibits" or "blocks" when used to describe a characteristic of the candidate compound of the present invention means a compound that when added at a concentration of about 10 µM in a standard chromogenic assay for FX activation (see, Roy, S., *J. Biol. Chem*. 266:4665-4668 (1991), O'Brien, D., *et al*., *J. Clin. Invest.* 82:206-212 (1988); Lee *et al*., *Biochemistry* 36:5607-5611 (1997); Kelly *et al*., *J. Biol. Chem.* 272:17467-17472 (1997)) produces at least a 50% inhibition of the conversion of Factor X to Factor Xa in the presence of Factor VII and other necessary reagents. Preferably the compound will produce at least a 50% inhibition at a concentration of about 1 µM and more preferably at least a 50% inhibition at a concentration of about 100 nM. In a more preferred embodiment the compound of the present invention will produce at least a 50% inhibition of the conversion of Factor X to Factor Xa when present in a concentration of about 10 nM or less.

### Peptides and Analogs Thereof

According to preferred aspects of the present invention the compound is a cyclic peptide or analog thereof. Preferably, the compound has the following formula:

Xᵢ-Cys₁-Xⱼ-Cys₂-Xₖ

wherein Xᵢ is absent or is a peptide of between 1 and 100 amino acids, preferably between about 1 and 50 amino acids, and more preferably between about 1 and 10; Xⱼ is 5 amino acids and Xₖ is absent or a peptide of between 1 and 100 amino acids, preferably between about 1 and 50 amino acids and more preferably between about 1 and 10, so long as the cyclic peptide or analog thereof retains the qualitative biological activity described above.

Preferred embodiments of the present invention include peptides such as those de'scribed above comprising the following sequence: Peptides within the scope of the invention compete with SEQ ID NO: 4 for binding FVII/FVIIa in an *in vitro* assay and have between 1 and 8 amino acids of SEQ ID NO: 4 substituted, preferably between 1 and 6 amino acids of SEQ ID NO: 4 substituted, more preferably between 1 and 4 amino acids, and more preferably 1 or 2 amino acids of SEQ ID NO: 4 substituted. According to this aspect of the invention Trp₁ is an amino acid selected from the group consisting of Trp, Phe, Tyr, Leu, Ile, Met, Val and Ala; Glu₁ is any amino acid; Val is an amino acid selected from the group consisting of Val, Trp, Phe, Tyr, Leu, Ile, Met and Ala; Leu is an amino acid selected from the group consisting of Leu, Trp, Phe, Tyr, Ile Met, Val and Ala; Trp₂ is amino acid selected from the group consisting of Trp, Phe, Tyr, Leu, Ile, Met, Val and Ala; Thr₁ is any amino acid; Trp₃ is an amino acid selected from the group consisting of Trp, Phe, Tyr, Leu, Ile, Met, Val and Ala; Glu₂ is any amino acid; Thr₂ is any amino acid; Glu₃ is any amino acid and Arg is an amino acid selected from the group consisting of Arg, Lys, Leu, Trp, His, Met and Ile.

Preferred amino acids according to this aspect of the invention comprise the sequence Trp₁-Glu₁-Val-Leu-Cys₁-Trp₂-Thr₁-Trp₃-Glu₂-Thr₂-Cys₂-Glu₃-Arg (SEQ ID NO:4) or compete with SEQ ID NO: 4 for binding FVII/FVIIa in an *in vitro* assay and having between 1 and 8 amino acids of SEQ ID NO: 4 substituted; more preferably between 1 and 6 amino acids of SEQ ID NO: 4 substituted, more preferably between 1 and 4 amino acids, and more preferably 1 or 2 amino acids of SEQ ID NO: 4 substituted. According to this aspect of the invention; Trp₁ is an amino acid selected from the group consisting of Trp, Phe and Leu; Glu₁ is any amino acid; Val is an amino acid selected from the group consisting of Val and Ile; Leu is an amino acid selected from the group consisting of Leu, Ile, Met, Val and Ala;Trp₂ is amino acid selected from the group consisting of Trp, Phe, Tyr, Leu and Met; Thr₁ is any amino acid; Trp₃ is an amino acid selected from the group consisting of Trp, Phe and Tyr; Glu₂ is any amino acid; Thr₂ is any amino acid; Glu₃ is any amino acid and Arg is an amino acid selected from the group consisting of Arg, Lys, Leu and Trp.

The foregoing peptides preferably have an IC₅₀ for FVII/FVIIa of less than 1 µM, more preferably less than 100 nM and more preferably less than 10 nM. In addition the peptides preferably binds FVII/FVIIa and inhibits activity associated with FVIIa selected from the group consisting of activation of FVII, activation of FIX and activation of FX. Preferably the peptide competes with a peptide of the present invention for binding FVII/FVIIa and blocks activation of FX. Preferably the peptide has an IC₅₀ for inhibiting FX activation of less than 10 µM, more preferably of less than 100 nM and more preferably less than 5 nM.

Preferred peptides of the present invention have the following formula:

Xᵢ-Cys₁-Xⱼ-Cys₂-Xₖ

wherein Xᵢ is absent or is between 1 and 100 amino acids; Xⱼ is 5 amino acids and Xₖ is absent or between 1 and 100 amino acids. Preferably, Xᵢ and Xₖ are between 1 and 50 amino acids and more preferably between 1 and 10 amino acids.

By way of exemplification and not limitation, prefered peptides of the present invention include the peptides described in Figure 4. In general, a preferred peptide has the formula:

Xᵢ-Cys₁-Xⱼ-Cys₂-Xₖ

wherein;
Xⱼ has the formula and Xaa₈ is an amino acid selected from the group consising of Trp, Thr, Ala, Phe, Leu, Met and Tyr; Xaa₉ is an amino acid selected from the group consisting of Thr, Asp and Ala; Xaa₁₀ is an amino acid selected from the group consisting of Trp, Ala, Phe, Leu and Tyr; Xaa₁₁ is an amino acid selected from the group consisting of Glu, Ala, Arg and Gln; and Xaa₁₂ is an amino acid selected from the group consisting of Gly, Asp, Thr, Ser and Ala.

As a further example, preferred peptides have the formula: wherein Xaa₁ is any amino acid; Xaa₂ is any amino acid; Xaa₃ is an amino acid selected from the group consisting of Trp, Phe, Leu, Ala, Met and Val; Xaa₄ is an amino acid; Xaa₅ is an amino acid selected from the group consisting of Val, Ile, Ala, Trp and Tyr; Xaa₆ is an amino acid selected from the group consisting of Leu, Ile, Met, Val and Ala; Xaa₈ is selected from the group consisting of Trp, Phe, Leu, Met, Ala and Val; Xaa₉ is an amino acid Xaa₁₀ is an amino acid selected from the group consisting of Trp, Phe, Met and Tyr; Xaa₁₁ is any amino acid; Xaa₁₂ is any amino acid; Xaa₁₄ is any amino acid except Pro; Xaa₁₅ is an amino acid selected from the group consisting of Arg, Lys, Leu, Trp, His and Met;
Xaa₁₆ is any amino acid; Xaa₁₇ is any amino acid; and Xaa₁₈ is any amino acid.

In this context reference can be made to the exemplary peptides listed in Figure 4. Preferred peptides are constructed according to the foregoing and considering the following amino acid selections: Xaa₃ is selected from the group consisting of Trp, Phe, Leu and Ala; Xaa₅ is selected from the group consisting of Val, Ile and Ala; and Xaa₇ is selected from the group consisting of Trp, Phe, Leu, Met and Ala. More preferably, the foregoing scheme is employed and amino acid selection are made as follows: Xaa₃ is selected from the group consisting of Trp, Phe and Leu; Xaa₅ is selected from the group consisting of Val and Ile; Xaa₆ is selected from the group consisting of Leu, Ile, Met and Val; Xaa₈ is selected from group consisting of Trp, Phe, Leu and Met; Xaa₁₀ is selected from the group consisting of Trp and Phe; and Xaa₁₅ is selected from the group consisting of Arg, Lys Leu and Trp.

The invention further provides a method of inhibiting FVIIa activity comprising the step of contacting FVII/FVIIa with a peptide of of the invention, such as those described above, in the presence of tissue factor and under conditions which allow binding of the compound to FVIIa to occur.

The invention also provides a method for selecting a compound which blocks FVII/FVIIa activation of FX comprising the step of:
Contacting FVII/FVIIa with a peptide of the invention in the presence and absence of a candidate molecule under conditions which allow specific binding of the peptide of the invention to FVII/FVIIa to occur; detecting the amount of specific binding of the peptide of the invention to FVII/FVIIa that occurs in the presence and absence of the candidate compound wherein the amount of binding in the presence of the candidate compound relative to the amount of binding in the absence of the candidate molecule is indicative of the ability of the candidate compound to block FVII/FVIIa activation of FX.

The invention further provides a method of inhibiting the activation of FX comprising contacting FVII/FVIIa with a compound that prevents the interaction of FVII/FVIIa with a peptide of the invention. The contacting step occurs *in vitro*.

The foregoing compounds can be used and are especially preferred in a method of inhibiting FVIIa activity comprising the steps of:
a) contacting FVIIa with a compound of interest, especially the peptides and peptide analogs described in the foregoing section, in the presence or absence of tissue factor and under conditions which allow binding of the compound to FVIIa to occur; and, optionally,
b) measuring or assessing the amount of FX activation that occurs in the presence of the compound of interest. According to certain aspects of the invention a standard FX activation assay as described herein is employed along with the foregoing method to measure the amount of FX activation that is blocked or inhibited by the compound of interest. This aspect of the present invention may be practiced *in vitro* or *in vivo*.

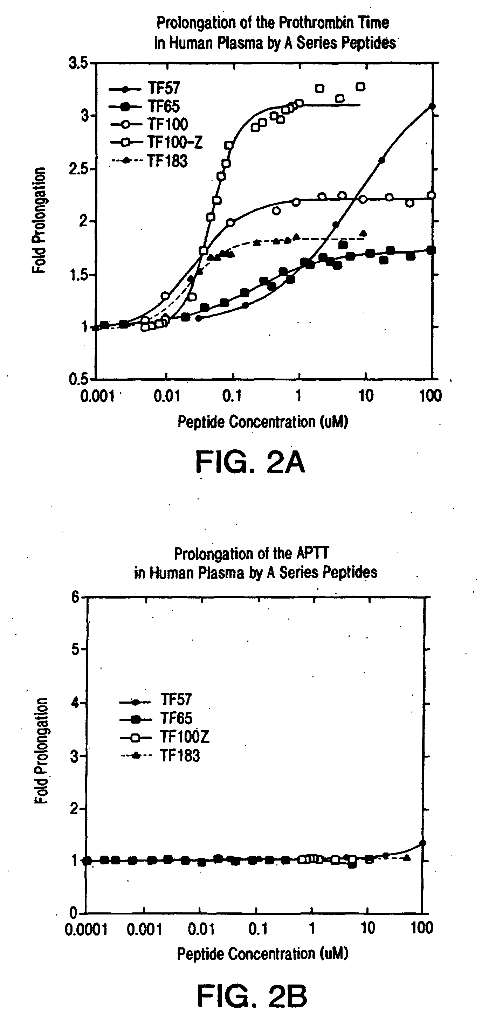

The sequences of the exemplary peptides (Figure 4) in combination with the information presented herein for dose dependent prolongation of prothrombin time (Figure 2A) along with the IC₅₀ values for inhibition of FX activation and FVIIa affinity (Figure 4) can be used in peptide design and selection. For example, the skilled artisan can employ the teachings of the invention to design a peptide which at saturating concentrations inhibits FX activation to varying degrees or prolongs the prothrombin time to varying degrees. For example, the skilled artisan may select a core peptide having, for example, the amino acid sequence of TF65 (SEQ ID NO: 4). N-terminal amino acid additions can be selected to improve the peptide's affinity for FVII/FVIIa while C-terminal amino acid additions can be selected to affect the degree of inhibition of FX activation or prolongation of the prothrombin time. Selecting TF65 (SEQ ID NO: 4) and adding 2 N-terminal Glu residues provides a peptide with a decreased IC₅₀ for FVIIa, as described in Figure 4 (SEQ ID NO: 23). Adding C-terminal amino acids, as for example -Gly-Glu-Gly to SEQ ID NO: 23, provides a peptide with improved affinity for FVIIa and increased ability to inhibit FX activation over SEQ ID NO: 4, as is seen with, for example TF100 (SEQ ID NO: 18) and TF100Z (SEQ ID NO: 19). Using this information the skilled artisan can construct peptides throughout a range of affinities and degrees of inhibition of FX activation and prolongation of the prothrombin time.

The ability to affect the degree of inhibition of FX activation and prolongation of the prothrombin time may prove useful in therapeutic areas where an anticoagulant would be useful, but .that overanticoagulation could prove clinically unsafe. Thus a peptide with the desired degree of inhibition of FX activation and prolongation of the prothrombin time may prove to be efficacious with the increased advantage of having a greater degree of safety such that any bleeding complications from overdosing would be minimized.

### Chemical Synthesis

One method of producing the compounds of the invention involves chemical synthesis. This can be accomplished by using methodologies well known in the art (see Kelley, R.F., and Winkler, M.E., in *Genetic Engineering Principles and Methods*, Setlow, J.K, ed., vol. 12, pp. 1-19 (Plenum Press, New York, New York, 1990); Stewart, J. M., and Young, J. D., *Solid Phase Peptide Synthesis* (Pierce Chemical Co., Rockford, Illinois, 1984); see also U.S. Patent Nos. 4,105,603; 3,972,859; 3,842,067; and 3,862,925).

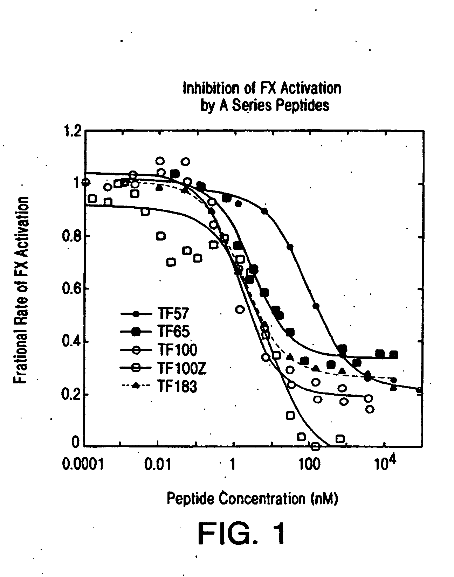

Peptides of the invention can be conveniently prepared using solid phase peptide synthesis (Merrifield, *J. Am. Chem. Soc.* 85:2149 (1964); Houghten, *Proc. Natl. Acad. Sci. USA* 82:5132 (1985)). Solid phase synthesis begins at the carboxyl terminus of the putative peptide by coupling a protected amino acid. to an inert solid support. The inert solid support can be any macromolecule capable of serving as an anchor for the C-terminus of the initial amino acid. Typically, the macromolecular support is a cross-linked polymeric resin (*e.g.*, a polyamide or polystyrene resin), as shown in Figures 1-1 and 1-2, on pages 2 and 4 of Stewart and Young, *supra.* In one embodiment, the C-terminal amino acid is coupled to a polystyrene resin to form a benzyl ester. A macromolecular support is selected such that the peptide anchor link is stable under the conditions used to deprotect the α-amino group of the blocked amino acids in peptide synthesis. If a base-labile α-protecting group is used, then it is desirable to use an acid-labile link between the peptide and the solid support. For example, an acid-labile ether resin is effective for base-labile Fmoc-amino acid peptide synthesis, as described on page 16 of Stewart and Young, *supra*. Alternatively, a peptide anchor link and α-protecting group that are differentially labile to acidolysis can be used. For example, an aminomethyl resin such as the phenylacetamidomethyl (Pam) resin works well in conjunction with Boc-amino acid peptide synthesis, as described on pages 11-12 of Stewart and Young, *supra*.

After the initial amino acid is coupled to an inert solid support, the α-amino protecting group of the initial amino acid is removed with, for example, trifluoroacetic acid (TFA) in methylene chloride and neutralizing in, for example, triethylamine (TEA). Following deprotection of the initial amino acid's α-amino group, the next α-amino and sidechain protected amino acid in the synthesis is added. The remaining α-amino and, if necessary, side chain protected amino acids are then coupled sequentially in the desired order by condensation to obtain an intermediate compound connected to the solid support. Alternatively, some amino acids may be coupled to one another to form a fragment of the desired peptide followed by addition of the peptide fragment to the growing solid phase peptide chain.

The condensation reaction between two amino acids, or an amino acid and a peptide, or a peptide and a peptide can be carried out according to the usual condensation methods such as the axide method, mixed acid anhydride method, DCC (N,N'-dicyclohexylcarbodiimide) or DIC (N,N'-diisopropylcarbodiimide) methods, active ester method, p-nitrophenyl ester method, BOP (benzotriazole-1-yl-oxy-tris [dimethylamino] phosphoniumhexafluorophosphate) method, N-hydroxysuccinic acid imido ester method, etc, and Woodward reagent K method.

It is common in the chemical syntheses of peptides to protect any reactive side-chain groups of the amino acids with suitable protecting groups. Ultimately, these protecting groups are removed after the desired polypeptide chain has been sequentially assembled. Also common is the protection of the α-amino group on an amino acid or peptide fragment while the C-terminal carboxyl group of the amino acid or peptide fragment reacts with the free N-terminal amino group of the growing solid phase polypeptide chain, followed by the selective removal of the α-amino group to permit the addition of the next amino acid or peptide fragment to the solid phase polypeptide chain. Accordingly, it is common in polypeptide synthesis that an intermediate compound is produced which contains each of the amino acid residues located in the desired sequence in the peptide chain wherein individual residues still carry side-chain protecting groups. These protecting groups can be removed substantially at the same time to produce the desired polypeptide product following removal from the solid phase.

α- and ε-amino side chains can be protected with benzyloxycarbonyl (abbreviated Z), isonicotinyloxycarbonyl (iNOC), o-chlorobenzyloxycarbonyl [Z(2Cl)], p-nitrobenzyloxycarbonyl [Z(NO₂)], p-methoxybenzyloxycarbonyl [Z(OMe)], t-butoxycarbonyl (Boc), t-amyloxycarbonyl (Aoc), isobornyloxycarbonyl, adamantyloxycarbonyl, 2-(4-biphenyl)-2-propyloxycarbonyl (Bpoc), 9-fluorenylmethoxycarbonyl (Fmoc), methylsulfonyethoxycarbonyl (Msc), trifluoroacetyl, phthalyl, formyl, 2-nitrophenylsulphenyl (NPS), diphenylphosphinothioyl (Ppt), and dimethylphosphinothioyl (Mpt) groups, and the like.

Protective groups for the carboxyl functional group are exemplified by benzyl ester (OBzl), cyclohexyl ester (Chx), 4-nitrobenzyl ester (ONb), t-butyl ester (Obut), 4-pyridylmethyl ester (OPic), and the like. It is often desirable that specific amino acids such as arginine, cysteine, and serine possessing a functional group other than amino and carboxyl groups are protected by a suitable protective group. For example, the guanidino group of arginine may be protected with nitro, p-toluenesulfonyl, benzyloxycarbonyl, adamantyloxycarbonyl, p-methoxybenzesulfonyl, 4-methoxy-2,6-dimethylbenzenesulfonyl (Nds), 1,3,5-trimethylphenysulfonyl (Mts), and the like. The thiol group of cysteine can be protected with p-methoxybenzyl, trityl, and the like.

Many of the blocked amino acids described above can be obtained from commercial sources such as Novabiochem (San Diego, California), Bachem CA (Torrence, California) or Peninsula Labs (Belmont, California).

Stewart and Young, *supra,* provides detailed information regarding procedures for preparing peptides. Protection of α-amino groups is described on pages 14-18, and side chain blockage is described on pages 18-28. A table of protecting groups for amine, hydroxyl and sulfhydryl functions is provided on pages 149-151.

After the desired amino acid sequence has been completed, the peptide can be cleaved away from the solid support, recovered and purified. The peptide is removed from the solid support by a reagent capable of disrupting the peptide-solid phase link, and optionally deprotects blocked side chain functional groups on the peptide. In one embodiment, the peptide is cleaved away from the solid phase by acidolysis with liquid hydrofluoric acid (HF), which also removes any remaining side chain protective groups. Preferably, in order to avoid alkylation of residues in the peptide (for example, alkylation of methionine, cysteine, and tyrosine residues), the acidolysis reaction mixture contains thio-cresol and cresol scavengers. Following HF cleavage, the resin is washed with ether, and the free peptide is extracted from the solid phase with sequential washes of acetic acid solutions. The combined washes are lyophilized, and the peptide is purified.

### Disulfide Linked Peptides

As described above, some embodiments of the invention are cyclized by formation of a disulfide bond between cysteine residues. Such peptides can be made by chemical synthesis as described above and then cyclized by any convenient method used in the formation of disulfide linkages. For example, peptides can be recovered from solid-phase synthesis with sulfhydryls in reduced form, dissolved in a dilute solution wherein the intramolecular cysteine concentration exceeds the intermolecular cysteine concentration in order to optimize intramolecular disulfide bond formation, such as a peptide concentration of 25 mM to 1 µM, and more preferably 500 µM to 1 µM, and more preferably 25 µM to 1 µM, and then oxidized by exposing the free sulfhydryl groups to a mild oxidizing agent that is sufficient to generate intramolecular disulfide bonds, *e.g.*, molecular oxygen with or without catalysts such as metal cations, potassium ferricyanide, sodium tetrathionate, *etc*. In one embodiment, the peptides are cyclized as described in Example 2 below. Alternatively, the peptides can be cyclized as described in Pelton *et al*., *J. Med*. *Chem*. 29:2370-2375 (1986).

Cyclization can be achieved by the formation, for example, of a disulfide bond or a lactam bond between Cys residues. Residues capable of forming a disulfide bond include for example Cys, Pen, Mpr, and Mpp and its 2-amino group-containing equivalents. Residues capable of forming a lactam bridge include, for example, Asp, Glu, Lys, Orn, αβ-diaminobutyric acid, diaminoacetic acid, aminobenzoic acid and mercaptobenzoic acid. The compounds herein can be cyclized, for example, via a lactam bond which can utilize the side chain group of a non-adjacent residue to form a covalent attachment to the N-terminus amino group of Cys or other amino acid. Alternative bridge structures also can be used to cyclize the compounds of the invention, including, for example, peptides and peptidomimetics, which can cyclize via S-S, CH2-S, CH2-O-CH2, lactam ester or other linkages.

### Recombinant Synthesis

In a further embodiment, the present invention encompasses a composition of matter comprising isolated nucleic acid, preferably DNA, encoding a peptide described herein. DNAs encoding the peptides of the invention can be prepared by a variety of methods known in the art. These methods include, but are not limited to, chemical synthesis by any of the methods described in Engels *et al*.,*Agnew. Chem. Int. Ed. Engl*. 28:716-734 (1989), the entire disclosure of which is incorporated herein by reference, such as the triester, phosphite, phosphoramidite and H-phosphonate methods. In one embodiment, codons preferred by the expression host cell are used in the design of the encoding DNA. Alternatively, DNA encoding the peptide can be altered to encode one or more variants by using recombinant DNA techniques, such as site-specific mutagenesis (Kunkel *et al*., *Methods Enzymol.* 204:125-139 (1991); Carter, P., *et al*., *Nucl. Acids. Res.* 13:4331 (1986); Zoller, M. J., *et al*., *Nucl. Acids Res*. 10:6487 (1982)), cassette mutagenesis (Wells, J. A., *et al*., *Gene* 34:315 (1985)), restriction selection mutagenesis (Wells, J. A., *et al*., *Philos. Trans, R. Soc*. *London*, SerA 317, 415), and the like.

The invention further comprises an expression control sequence operably linked to the DNA molecule encoding a peptide of the invention, and an expression vector, such as a plasmid, comprising the DNA molecule, wherein the control sequence is recognized by a host cell transformed with the vector. In general, plasmid vectors contain replication and control sequences which are derived from species compatible with the host cell. The vector ordinarily carries a replication site, as well as sequences which encode proteins that are capable of providing phenotypic selection in transformed cells.

Suitable host cells for expressing the DNA include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E*. *coli* K12 strain MM294 (ATCC No. 31,446); *E. coli* X1776 (ATCC No. 31,537); *E. coli* strain W3110 (ATCC No. 27,325) and K5 772 (ATCC No. 53,635).

In addition to prokaryotes, eukaryotic organisms, such as yeasts, or cells derived from multicellular organisms can be used as host cells. For expression in yeast host cells, such as common baker's yeast or *Saccharomyces cerevisiae*, suitable vectors include episomally replicating vectors based on the 2-micron plasmid, integration vectors, and yeast artificial chromosome (YAC) vectors. Suitable host cells for expression also are derived from multicellular organisms. Examples of invertebrate cells include insect cells such as Drosophila S2 and Spodoptera Sf9, as well as plant cells. For expression in insect host cells, such as Sf9 cells, suitable vectors include baculoviral vectors. For expression in plant host cells, particularly dicotyledonous plant hosts, such as tobacco, suitable expression vectors include vectors derived from the Ti plasmid of *Agrobacterium tumefaciens*.

Examples of useful mammalian host cells include monkey kidney CV1 line transformed by SV40 (COS-7, ATCC CRL 1651); human embryonic kidney line (293 or 293 cells subcloned for growth in suspension culture, Graham *et al*., *J. Gen Virol*. 36:59 (1977)); baby hamster kidney cells (BHK, ATCC CCL 10); Chinese hamster ovary cells/-DHFR (CHO, Urlaub and Chasin, *Proc. Natl. Acad. Sci. USA* 77:4216 (1980)); mouse sertoli cells (TM4, Mather, *Biol. Reprod.* 23:243-251 (1980)); monkey kidney cells (CV1 ATCC CCL 70);.African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather *et al*., *Annals N.Y. Acad. Sci*. 383:44-68 (1982)); MRC 5 cells; FS4 cells; and a human hepatoma cell line (Hep G2).

For expression in prokaryotic hosts, suitable vectors include pBR322 (ATCC No. 37,017), phGH107 (ATCC No. 40,011), pB0475, pS0132, pRIT5, any vector in the pRIT20 or pRIT30 series (Nilsson and Abrahmsen, *Meth. Enzymol*. 185:144-161 (1990)), pRIT2T, pKK233-2, pDR540 and pPL-lambda. Prokaryotic host cells containing the expression vectors of the present invention include *E. coli* K12 strain 294 (ATCC NO. 31,446), *E*. *coli* strain JM101 (Messing *et al*., *Nucl. Acid Res*. 9:309 (1981)), *E. coli* strain B, *E. coli* strain χ1776 (ATCC No. 31,537), *E. coli* c600 (Appleyard, Genetics 39:440 (1954)), *E. coli* W3110 (F-, gamma-, prototrophic, ATCC No. 27,325), *E. coli* strain 27C7 (W3110, *tonA, phoA E15, (argF-lac)169, ptr3, degP41, ompT, kan*^{r}) (U.S. Patent No. 5,288,931, ATCC. No. 55,244), *Bacillus subtilis, Salmonella typhimurium, Serratia marcesans,* and *Pseudomonas* species.

For expression in mammalian host cells, useful vectors include vectors derived from SV40, vectors derived from cytomegalovirus such as the pRK vectors, including pRK5 and pRK7 (Suva *et al*., *Science* 237:893-896 (1987); EP 307,247 (3/15/89), EP 278,776 (8/17/88)) vectors derived from vaccinia viruses or other pox viruses, and retroviral vectors such as vectors derived from Moloney's murine leukemia virus (MoMLV).

Optionally, the DNA encoding the peptide of interest is operably linked to a secretory leader sequence resulting in secretion of the expression product by the host cell into the culture medium. Examples of secretory leader sequences include stII, ecotin, lamB, herpes GD, lpp, alkaline phosphatase, invertase, MIP.5 and alpha factor. Also suitable for use herein is the 36 amino acid leader sequence of protein A (Abrahmsen *et al*., *EMBO J*. 4:3901 (1985)).

Host cells are transfected and preferably transformed with the above-described expression or cloning vectors of this invention and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ precipitation and electroporation. Successful transfection is generally recognized when any indication of the operation of this vector occurs within the host cell.

Transformation means introducing DNA into an organism so that the DNA is replicable, either as an extrachromosomal element or by chromosomal integrant. Depending upon the host cell used, transformation is done using standard techniques appropriate to such cells. The calcium treatment employing calcium chloride, as described in section 1.82 of Sambrook *et al.*, *Molecular Cloning,* 2nd ed. (Cold Spring Harbor Laboratory, New York, 1989), is generally used for prokaryotes or other cells that contain substantial cell-wall barriers. Infection with *Agrobacterium tumefaciens* is used for transformation of certain plant cells, as described by Shaw *et al*., *Gene* 23:315 (1983) and WO 89/05859, published 29 June 1989. For mammalian cells without such cell walls, the calcium phosphate precipitation method described in sections 16.30-16.37 of Sambrook *et al*., *supra*, is preferred. General aspects of mammalian cell host system transformations have been described by Axel in U.S. Patent No. 4,399,216, issued 16 August 1983. Transformations into yeast are typically carried out according to the method of Van Solingen *et al*., *J. Bact*. 130:946 (1977) and Hsiao *et al*., *Proc. Natl*. *Acad. Sci. USA* 76:3829 (1979). However, other methods for introducing DNA into cells such as by nuclear injection, electroporation, or by protoplast fusion may also be used.

Other preferred vectors can be constructed using standard techniques.by combining the relevant traits of the vectors described above. Relevant traits include the promoter, the ribosome binding site, the gene of interest or gene fusion (the Z domain of protein A and gene of interest and a linker), the antibiotic resistance markers, and the appropriate origins of replication.

A variation on the above procedures contemplates the use of gene fusions, wherein the gene encoding the desired peptide is associated, in the vector, with a gene encoding another protein or a fragment of another protein. This results in the desired peptide being produced by the host cell as a fusion with another protein or peptide. The "other" protein or peptide is often a protein or peptide which can be secreted by the cell, making it possible to isolate and purify the desired peptide from the culture medium and eliminating the necessity of destroying the host cells which arises when the desired peptide remains inside the cell. Alternatively, the fusion protein can be expressed intracellularly. It is useful to use fusion proteins that are highly expressed.

The use of gene fusions, though not essential, can facilitate the expression of heterologous peptides in insect cells as well as the subsequent purification of those gene products. Protein A fusions are often used because the binding of protein A, or more specifically the Z domain of protein A,'to IgG provides an "affinity handle" for the purification of the fused protein. For example, a DNA sequence encoding the desired peptide ligand can be fused by site-directed mutagenesis to the genen for a consensus domain of protein A known as the Z domain (Nilsson *et al*., *Protein Engineering* 1:107-113 (1987)). After expression and secretion the fusion protein can be enzymatically cleaved to yield free peptide which can be purified from the enzymatic mix (see, *e.g*., Varadarajan *et al*., *Proc. Natl. Acad. Sci USA* 82:5681-5684 (1985); Castellanos-Serra *et al*., *FEBS Letters* 378:171-176 (1996); Nilsson *et al*., *J. Biotechnol*. 48:241-250 (1996)).

Fusion proteins can be cleaved using chemicals, such as cyanogen bromide, which cleaves at a methionine, or hydroxylamine, which cleaves between an Asn and Gly residue. Using standard recombinant DNA methodology, the nucleotide base pairs encoding these amino acids may be inserted just prior to the 5' end of the gene encoding the desired peptide.

Alternatively, one can employ proteolytic cleavage of fusion protein. Carter, in *Protein Purification: From Molecular Mechanisms to Large-Scale Processes,* Ladisch *et al*., eds., Ch. 13, pp. 181-193 (American Chemical Society Symposium Series No. 427, 1990).

Proteases such as Factor Xa, thrombin, and subtilisin or its mutants, and a number of others have been successfully used to cleave fusion proteins. Preferred according to the present invention for the production of peptide ligands of less than about 30 amino acids is the protease trypsin which is highly specific for Arg and Lys residues. Trypsin cleavage is discussed generally in Nilsson *et al*., *J*. *Biotech*. 48:241 (1996) and Smith *et al*., *Methods Mol. Biol*. 32:289 (1994). Typically, a peptide linker that is amenable to cleavage by the protease used is inserted between the "other" protein (*e.g*., the Z domain of protein A) and the desired peptide. Using recombinant DNA methodology, the nucleotide base pairs encoding the linker are inserted between the genes or gene fragments coding for the other proteins. Proteolytic cleavage of the partially purified fusion protein containing the correct linker can then be carried out on either the native fusion protein, or the reduced or denatured fusion protein.

The peptide may or may not be properly folded when expressed as a fusion protein. Also, the specific peptide linker containing the cleavage site may or may not be accessible to the protease. These factors determine whether the fusion protein must be denatured and refolded, and if so, whether these procedures are employed before or after cleavage.

When denaturing and refolding are needed, typically the peptide is treated with a chaotrope, such a guanidine HCl, and is then treated with a redox buffer, containing, for example, reduced and oxidized dithiothreitol or glutathione at the appropriate ratios, pH, and temperature, such that the peptide is refolded to its native structure.

The host cells referred to in this disclosure encompass cells in *in vitro* culture as well as cells that are within a host animal.

In cyclized embodiments of the invention, the recombinantly produced peptide can be cyclized by formation of an intramolecular disulfide bond as described above.

The peptide compounds of the invention can be modified at the N-terminus or the C-terminus using an amino-protecting group or carboxyl-protecting group, respectively. Numerous such modifications will be apparent to those skilled in the art. For example, the N-terminus of a peptide or peptide analog can be chemically modified such that the N-terminal amino group is substituted for example by an acetyl, cyclopentylcarboxy, isoquinolylcarboxy, furoyl, tosyl, pyrazinecarboxy, or other such group, which can be sustituted by a substituent as described herein. The N-terminal amino group also can be substituted, for example, with a reverse amide bond. It should be recognized that the term amino group is used broadly herein to refer to any free amino group, including a primary, secondary, or tertiary amino group, present in a peptide. By contrast the term N-terminus refers to the α-amino group of the first amino acid present in a peptide written in the conventional manner.

The N-terminus of a peptide of the invention can be protected by linking thereto an amino protecting group. The term "amino protecting group" is used broadly herein to refer to a chemical group that can react with a free amino group, including, for example, the α-amino group present at the N-terminus of an peptide of the invention. By virtue of reacting therewith, an amino protecting group protects the otherwise reactive amino group against undesirable reactions, as can occur, for example, during a synthetic procedure or due to exopeptidase activity on a final compound.

Modification of an amino group also can provide additional advantages, including, for example, increasing the solubility or the activity of the compound. Compounds having these modifications are meant to be included within the compounds of the present invention since their construction is within the ability of the skilled artisan given the present disclosure. Various amino protecting groups are known in the art and include, for example, acyl groups such as an acetyl, picolyl, tert-butylacetyl, tert-butyloxycarbonyl, benzyloxycarbonyl, benzoyl groups, including for example a benzyloxime such as a 2-aryl-2-o-benzyloxime as well as an amino acyl residue which itself can be modified by an amino-protecting group. Other amino-protecting groups are described, for example, in *The Peptides,* Gross and Meienhofer, eds., Vol. 3 (Academic Press, Inc., New York, New York, 1981) and Greene and Wuts, in *Protective groups in Organic Synthesis,* 2d ed., pp. 309-405 (John Wiley & sons, New York, New York, 1991), each of which is incorporated herein by reference. The product of any such modification of the N-terminus amino group of a peptide or peptide analog of the invention is referred to herein as an "N-terminal derivative".

Similarly, a carboxyl group such as the carboxyl group present at the C-terminus of a peptide can be chemically modified using a carboxyl-protecting group. The terms "carboxyl group" and "C-terminus" are used in a manner consistent with the terms amino groups and N-terminus as defined above. A carboxyl group such as that present at the C-terminus of a peptide can be modified by reduction of the C-terminal carboxyl group to an alcohol or aldehyde or by formation of an oral ester or by substitution of the carboxyl group with a substituent such as a thiazolyl, cyclohexyl, or other group. Oral esters are well known in the art and include, for example, alkoxymethyl groups such as methoxymethyl, ethoxymethyl, propoxymethyl, isopropoxy methyl, and the like.

### Peptide Combinations

### A. Multimerization domains

According to a preferred embodiment of the invention, the peptide compounds are combined with a multimerization domain. According to this aspect of the invention, hybrid molecules are provided which comprise at least two distinct domains. Each molecule comprises a peptide domain and a multimerization domain. According to the present invention, the peptide domain is joined to a multimerization domain such as an immunoglobulin Fc region, optionally via a flexible linker domain.

The hybrid molecules of the present invention are constructed by combining the peptide with a suitable multimerization domain. Ordinarily, when preparing the hybrid molecules of the present invention, nucleic acid encoding the peptide will be operably linked to nucleic acid encoding the multimerization domain sequence. Typically the construct encodes a fusion protein wherein the C-terminus of the peptide is joined to the N-terminus of the multimerization domain. However, fusions where, for example, the N-terminus of the peptide is joined to the C-terminus of the multimerization domain are also possible.

Preferred multimerization domains are immunoglobulin constant region sequences. Typically, in such fusions the encoded hybrid molecule will retain at least functionally active hinge, CH2 and CH3 domains of the constant region of an immunoglobulin heavy chain. Fusions are also made, for example, to the C-terminus of the Fc portion of a constant domain, or immediately N-terminal to the CH1 of the heavy chain or the corresponding region of the light chain.

The precise amino acid site at which the fusion of the peptide to the immunoglobulin constant domain is made is not critical; particular sites are well known and may be selected in order to optimize the biological activity, secretion, or binding characteristics. In this regard, the skilled artisan may reference the construction of various immunoadhesins described in the literature (U.S. Patent Nos. 5,116,964, 5,714,147 and 5,336,603; Capon et al., *Nature* 337:525-531 (1989); Traunecker *et al*., *Nature* 339:68-70 (1989); and Byrn *et al*., *Nature* 344:667-670 (1990); Watson *et al*., *J. Cell. Biol*. 110:2221-2229 (1990); Watson *et al*., *Nature* 349:164-167 (1991); Aruffo *et al*., *Cell* 61:1303-1313 (1990); Linsley *et al*., *J. Exp. Med*. 173:721-730 (1991); Linsley *et al*., *J. Exp. Med.* 174:561-569 (1991); Stamenkovic *et al*., *Cell* 66:1133-1144 (1991); Ashkenazi *et al*., *Proc. Natl. Acad. Sci. USA* 88:10535-10539 (1991); Lesslauer *et al*., *Eur. J. Immunol*. 27:2883-2886 (1991); Peppel *et al*., *J. Exp. Med.* 174:1483-1489 (1991); Mohler *et al*., *J. Immunol.* 151:1548-1561 (1993); Bennett *et al*., *J. Biol. Chem*. 266:23060-23067 (1991); Kurschner *et al*., *J. Biol. Chem.* 267:9354-9360 (1992); Chalupny *et al*., *PNAS USA* 89:10360-10364 (1992); Ridgway and Gorman, *J. Cell. Biol.* 115, Abstract No. 1448, (1991)).

According to a particular aspect, an immunoglobulin type multimerization domain is selected to provide a multimer such as a dimer having an immunoglobulin Fc region. Therefore, the peptide is joined, in particular aspects, to an immunoglobulin heavy chain constant domain to provide a multimer comprising a functional Fc domain. In this case, DNA encoding an immunoglobulin chain-peptide sequence is typically coexpressed with the DNA encoding a second peptide-immunoglobulin heavy chain fusion protein. Upon secretion, the hybrid heavy chain will be covalently associated to provide an immunoglobulin-like structure comprising two disulfide-linked immunoglobulin heavy chains.

Preferably, the Fc region is a human Fc region, *e.g.*, a native sequence human Fc region human IgG1 (A and non-A allotypes), IgG2, IgG3 or IgG4 Fc region.

In a preferred embodiment, the peptide sequence is fused to the N-terminus of the Fc region of immunoglobulin G₁ (IgG₁). It is possible to fuse the entire heavy chain constant region to the peptide sequence. However, more preferably, a sequence beginning in the hinge region just upstream of the papain cleavage site which defines IgG Fc chemically (*i.e.*, residue 216, taking the first residue of heavy chain constant region to be 114), or analogous sites of other immunoglobulins is used in the fusion. In a particularly preferred embodiment, the peptide amino acid sequence is fused to (a) the hinge region and CH2 and CH3 or (b) the CH1, hinge, CH2 and CH3 domains, of an IgG heavy chain; in a preferred embodiment the peptide ligand amino acid sequence is fused to (a) the hinge region and (b) the CH3 domain of IgG1.

According to a particular aspect of this embodiment, hybrid molecules comprising a peptide and a multimerization domain are assembled as multimers, for example homodimers, or heterodimers or even heterotetramers. Homodimers result from the pairing or crosslinking of two monomers comprising a peptide and a multimerization domain. However, it is not essential that two identical monomers pair. According to a particular aspect of the invention, a hybrid molecule as defined herein comprising a peptide and a multimerization domain such as an immunoglobulin constant domain may pair with a companion immunoglobulin chain comprising one arm of an immunoglobulin. Various exemplary assembled hybrid molecules within the scope of the present invention are schematically diagramed below:
(a) ACH
(b) ACH-ACH
(c) ACH-VHCH-VLCL
(d) ACH-VHCH
wherein each A represents identical or different peptide;
VL is an immunoglobulin light chain variable domain;
VH is an immunoglobulin heavy chain variable domain;
CL is an immunoglobulin light chain constant domain and
CH is an immunoglobulin heavy chain constant domain.

The hybrid molecules described herein are most conveniently constructed by fusing the cDNA sequence encoding the peptide portion in-frame to an immunoglobulin cDNA sequence. However, fusion to genomic immunoglobulin fragments can also be used (see, *e.g*., Aruffo *et al*., *Cell* 61:1303-1313 (1990); and Stamenkovic *et al*., *Cell* 66:1133-1144 (1991)). The latter type of fusion requires the presence of Ig regulatory sequences for expression. cDNAs encoding IgG heavy-chain constant regions can be isolated based on published sequences from cDNA libraries derived from spleen or peripheral blood lymphocytes, by hybridization or by polymerase chain reaction (PCR) techniques. The cDNAs encoding the peptides and the immunoglobulin parts of the hybrid molecule are inserted in tandem into a plasmid vector that directs efficient expression in the chosen host cells.

Alternatively, and especially in embodiments where the peptide is synthesized by, for example standard solid phase synthesis techniques, the peptide may be linked to the multimerization domain by any of a variety of means familiar to those of skill in the art. Covalent attachment is typically the most convenient, but other forms of attachment may be employed depending upon the application. Examples of suitable forms of covalent attachment include the bonds resulting from the reaction of molecules bearing activated chemical groups with amino acid side chains in the multimerization domain and can be made using a variety of bifunctional protein coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene).

### Peptide Fusions

According to the present invention, the peptide is optionally linked to, for example, another peptide either directly or via a flexible peptide linker. According to the present invention, the linker domain is any group of molecules that provides a spatial bridge between two or more peptide domains, as described in more detail herein below. According to this aspect of the invention, peptides are linked together, as, for example, in a fusion protein.

### Linker Domains

According to the present invention, the peptide domain is optionally linked to, for example, another peptide domain or a multimerization domain via a flexible peptide linker. The linker component of the hybrid molecule of the invention does not necessarily participate in but may contribute to the function of the hybrid molecule. Therefore, according to the present invention, the linker domain is any group of molecules that provides a spatial bridge between two or more peptide domains or a peptide domain and a multimerization domain.

The linker domain can be of variable length and makeup. It is, generally, the length of the linker domain and not its structure that is important. The linker domain preferably allows for the peptide domain of the hybrid molecule to bind, substantially free of spacial/conformational restrictions to the coordinant FVII/FVIIa molecule. Therefore, the length of the linker domain is dependent 'upon the character of the two functional domains; *e.g.*, the peptide and the multimerization domains of the hybrid molecule.

One skilled in the art will recognize that various combinations of atoms provide for variable-length molecules based upon known distances between various bonds (Morrison and Boyd, *Organic Chemistry,* 3rd Ed. (Allyn and Bacon, Inc., Boston, Massachusetts, (1977)). For example, the linker domain may be a polypeptide of variable length. The amino acid composition of the polypeptide determines the character and length of the linker. Exemplary linker domains comprise one or more Gly and or Ser/Arg residues.

### Research and Diagnostic Compositions

In a preferred embodiment, the peptides of the invention are noncovalently adsorbed or covalently bound to a macromolecule, such as a solid support. It will be appreciated that the invention encompasses both macromolecules complexed with the peptides. In general, the solid support is an inert matrix, such as a polymeric gel, comprising a three-dimensional structure, lattice or network of a material. Almost any macromolecule, synthetic or natural, can form a gel in a suitable liquid when suitably cross-linked with a bifunctional reagent. Preferably, the macromolecule selected is convenient for use in affinity chromatography. Most chromatographic matrices used for affinity chromatography are xerogels. Such gels shrink on drying to a compact solid comprising only the gel matrix. When the dried xerogel is resuspended in the liquid, the gel matrix imbibes liquid, swells and returns to the gel state. Xerogels suitable for use herein include polymeric gels, such as. cellulose, cross-linked dextrans (*e.g.*, Sepharose), agarose, cross-linked agarose, polyacrylamide gels, and polyacrylamide-agarose gels.

Alternatively, aerogels can be used for affinity chromatography. These gels do not shrink on drying but merely allow penetration of the surrounding air. When the dry gel is exposed to liquid, the latter displaces the air in the gel. Aerogels suitable for use herein include porous glass and ceramic gels.

Also encompassed herein are the peptides of the invention coupled to derivatized gels wherein the derivative moieties facilitate the coupling of the peptide ligands to the gel matrix and avoid steric hindrance of the peptide-FVII/FVIIa interaction in affinity chromatography. Alternatively, spacer arms can be interposed between the gel matrix and the peptide ligand for similar benefits.

In another embodiment, the invention provides fusion proteins in which a selected or desired polypeptide is fused at its N-terminus or its C-terminus, or at both termini, to one or more of the present peptides.

### Pharmaceutical Compositions

Pharmaceutical compositions which comprise the compounds, including the hybrid molecules of the invention; may be formulated and delivered or administered in a manner best suited to the particular FVII/FVIIa mediated disease or disorder being treated., including formulations suitable for parental, topical, oral, local, aerosol or transdermal administration or delivery of the compounds. In indications where the reduction of TF-FVIIa dependent coagulation is related to circulation of blood through stents or artificial valves or related to extracorporeal circulation, including blood removed in-line from a patient in such processes as dialysis procedures, blood filtration, or blood bypass during surgery, suitable formulations include those appropriate for coating devices such as stents, valves and filtration devices.

Somewhat more particularly, suitable compositions of the present invention comprise any of the compounds described herein along with a pharmaceutically acceptable carrier, the nature of the carrier differing with the mode of administration delivery or use, for example, in oral administration, usually using a solid carrier 'and in i.v. administration, a liquid salt solution carrier. For local administration, such as may be appropriate where TF-FVIIa dependent coagulation is related to circulation of blood through artificial devices such as stents or valves, the peptides may be linked, for example, covalently, to the artificial device preventing local thrombus formation. Alternatively, the peptide may be provided in a formulation that would allow for the peptide to slowly elute from the device providing both local and systemic protection against events associated with TF-FVIIa dependent coagulation. As but one example, stents adsorbed with peptides can be employed following angioplasty or other surgical procedure.

The compositions of the present invention include pharmaceutically acceptable components that are compatible with the subject and the compound of the invention. These generally include suspensions, solutions and elixirs, and most especially biological buffers, such as phosphate buffered saline, saline, Dulbecco's Media, and the like. Aerosols may also be used, or carriers such as starches, sugars, microcrystalline cellulose, diluents, granulating agents, lubricants, binders, disintegrating agents, and the like (in the case of oral solid preparations, such as powders, capsules, and tablets).

As used herein, the term "pharmaceutically acceptable" generally means approved by a regulatory agency of the Federal or a state government or listed in the U.S. Pharmacopeia or other generally recognized pharmacopeia for use in animals, and more particularly in humans.

The formulation of choice can be made using a variety of the aforementioned buffers, or even excipients including, for example, pharmaceutical grades of mannitol, lactose, starch, magnesium stearate, sodium saccharin cellulose, magnesium carbonate, and the like. "PEGylation" of the compositions may be achieved using techniques known to the art (see for example International Patent Publication No. WO92/16555, U.S. Patent No. 5,122,614 to Enzon, and International Patent Publication No. WO92/00748).

A preferred route of administration of the present invention is in the aerosol or inhaled form. The compounds of the present invention, combined with a dispersing agent, or dispersant, can be administered in an aerosol formulation as a dry powder or in a solution or suspension with a diluent.

As used herein, the term "dispersant" refers to a agent that assists aerosolization of the compound or absorption of the protein in lung tissue, or both. Preferably the dispersant is pharmaceutically acceptable. As used herein, the term "pharmaceutically acceptable" means approved by a regulatory agency of the Federal or a state government or listed in the U.S. Pharmacopeia or other generally recognized pharmacopeia for use in animals, and more particularly in humans. Suitable dispersing agents are well known in the art, and include, but are not limited to, surfactants and the like. For example, surfactants that are generally used in the art to reduce surface-induced aggregation of the compound, especially the peptide compound, caused by atomization of the solution forming the liquid aerosol may be used. Nonlimiting examples of such surfactants include polyoxyethylene fatty acid esters and alcohols, and polyoxyethylene sorbitan fatty acid esters. Amounts of surfactants used will vary, being generally within the range of 0.001 and 4% by weight of the formulation. In a specific aspect, the surfactant is polyoxyethylene sorbitan monooleate or sorbitan trioleate. Suitable surfactants are well known in the art, and can be selected on the basis of desired properties, depending upon the specific formulation, concentration of the compound, diluent (in a liquid formulation) or form of powder (in a dry powder formulation), *etc*.

Moreover, depending upon the choice of the compound, the desired therapeutic effect, the quality of the lung tissue (*e.g.*, diseased or healthy lungs), and numerous other factors, the liquid or dry formulations can comprise additional components, as discussed further below.

The liquid aerosol formulations generally contain the compound and a dispersing agent in a physiologically acceptable diluent. The dry powder aerosol formulations of the present invention consist of a finely divided solid form of the compound and a dispersing agent. With either the liquid or dry powder aerosol formulation, the formulation must be aerosolized. That is, it must be broken down into liquid or solid particles in order to ensure that the aerosolized dose actually reaches the alveoli. In general, the mass median dynamic diameter will be 5 micrometers or less in order to ensure that the drug particles reach the lung alveoli (Wearley, L. L., *Crit. Rev. in Ther. Drug Carrier Systems* 8:333 (1991)). The term "aerosol particle" is used herein to describe the liquid or solid particle suitable for pulmonary administration, *i.e.*, that will reach the alveoli. Other considerations such as construction of the delivery device, additional components in the formulation, and particle characteristics are important. These aspects of pulmonary administration of a drug are well known in the art, and manipulation of formulations, aerosolization means and construction of a delivery device require, at most, routine experimentation by one of ordinary skill in the art.

With regard to construction of the delivery device, any form of aerosolization known in the art, including, but not limited to, nebulization, atomization or pump aerosolization of a liquid formulation, and aerosolization of a dry powder formulation, can be used in the practice of the invention. A delivery device that is uniquely designed for administration of solid formulations is envisioned. Often, the aerosolization of a liquid or a dry powder formulation will require a propellent. The propellent may be any propellant generally used in the art. Specific nonlimiting examples of such useful propellants are a chlorofluorocarbon, a hydrofluorocarbon, a hydrochlorofluorocarbon, or a hydrocarbon, including trifluoromethane, dichlorodifluoromethane, dichlorotetrafluoroethanol, and 1,1,1,2-tetrafluoroethane, or combinations thereof.

In a preferred aspect of the invention, the device for aerosolization is a metered dose inhaler. A metered dose inhaler provides a specific dosage when administered, rather than a variable dose depending upon administration. Such a metered dose inhaler can be used with either a liquid or a dry powder aerosol formulation. Metered dose inhalers are well known in the art.

Once the compound reaches the lung, a number of formulation-dependent factors effect the drug absorption. It will be appreciated that in treating a disease or disorder that requires circulatory levels of the compound, such factors as aerosol particle size, aerosol particle shape, the presence or absence of infection, lung disease or emboli may affect the absorption of the compounds. For each of the formulations described herein, certain lubricators, absorption enhancers, protein stabilizers or suspending agents may be appropriate. The choice of these additional agents will vary depending upon the goal. It will be appreciated that in instances where local delivery of the compounds is desired or sought, such variables as absorption enhancement will be less critical.

### Liquid Aerosol Formulations

The liquid aerosol formulations of the present invention will typically be used with a nebulizer. The nebulizer can be either compressed-air driven or ultrasonic. Any nebulizer known in the art can be used in conjunction with the present invention, such as, but not limited to: Ultravent, (Mallinckrodt, Inc., St. Louis, Missouri); the Acorn II nebulizer (Marquest Medical Products, Englewood Colorado). Other nebulizers useful in conjunction with the present invention are described in U.S. Patent Nos. 4,624,251, issued November 25, 1986; 3,703,173, issued November 21, 1972; 3,561,444, issued February 9, 1971; and 4,635,627, issued January 13, 1971.

The formulation may include a carrier. The carrier is a macromolecule which is soluble in the circulatory system and which is physiologically acceptable where physiological acceptance means that those of skill in the art would accept injection of said carrier into a patient as part of a therapeutic regime. The carrier preferably is relatively stable in the circulatory system with an acceptable plasma half life for clearance. Such macromolecules include, but are not limited to, Soya lecithin, oleic acid and sorbitan trioleate, with sorbitan trioleate preferred.

The formulations of the present embodiment may also include other agents useful for protein stabilization or for the regulation of osmotic pressure. Examples of the agents include, but are not limited to, salts, such as sodium chloride, or potassium chloride, and carbohydrates, such as glucose, galactose or mannose, and the like.

### Aerosol Dry Powder Formulations

It is also contemplated that the present pharmaceutical formulation will be used as a dry powder inhaler formulation comprising a finely divided powder form of the compound and a dispersant. The form of the compound will generally be a lyophilized powder. Lyophilized forms of peptide compounds can be obtained through standard techniques.

In another embodiment, the dry powder formulation will comprise a finely divided dry powder containing one or more compounds of the present invention, a dispersing agent and also a bulking agent. Bulking agents useful in conjunction with the present formulation include such agents as lactose, sorbitol, sucrose, or mannitol, in amounts that facilitate the dispersal of the powder from the device.

### Therapeutic Methods

The compounds of the present invention can be used in preparation of a medicament to prevent the biological activity of the TF-FVIIa complex. The inhibition of TF-FVIIa is desirable in indications where the reduction of TF-FVIIa dependent coagulation is implicated. These situations include but are not limited to the prevention of arterial thrombosis in combination with thrombolytic therapy. It has been suggested that the TF-FVIIa plays a significant role in a variety of clinical states including deep venous thrombosis, arterial thrombosis, stroke, DIC, septic shock, cardiopulmonary bypass surgery, adult respiratory distress syndrome, hereditary angioedema. Inhibitors of TF-FVIIa may therefore play important roles in the regulation of inflammatory and/or thrombotic disorders.

Thus the present invention encompasses use of the compounds of the present invention for preparation of a medicament for preventing TF-FVIIa mediated event in a human comprising administering to a patient in need thereof a therapeutically effective amount of the compound of the present invention. A therapeutically effective amount of the compound of the present invention is predetermined to achieve the desired effect. The amount to be employed therapeutically will vary depending upon therapeutic objectives, the routes of administration and the condition being treated. Accordingly, the dosages to be administered are sufficient to bind to available FVII/FVIIa and form an inactive complex leading to decreased coagulation in the subject being treated.

The therapeutic effectiveness is measured by an improvement in one or more symptoms associated with the TF-FVIIa dependant coagulation. Such therapeutically effective dosages can be determined by the skilled artisan and will vary depending upon the age, sex and condition of the subject being treated. Suitable dosage ranges for systemic administration are typically between about 1 µg/kg to up to 100 mg/kg or more and depend upon the route of administration. According to the present invention, a preferred therapeutic dosage is between about 1 µg/kg body weight and about 5 mg/kg body weight. For example, suitable regimens include intravenous injection or infusion sufficient to maintain concentration in the blood in the ranges specified for the therapy contemplated.

The conditions characterized by abnormal thrombosis include those involving the arterial and venous vasculature. With respect to the coronary arterial vasculature, abnormal thrombus formation characterizes, for example, the rupture of an established atherosclerotic plaque which is the major cause of acute myocardial infarction and unstable angina, as well as also characterizing the occlusive coronary thrombus formation resulting from either thrombolytic therapy or percutaneous transluminal coronary angioplasty (PTCA). With respect to the venous vasculature, abnormal thrombus formation characterizes the condition observed in patients undergoing surgery in the lower extremities or the abdominal area who often suffer from thrombus formation in the venous vasculature resulting in reduced blood flow to the affected extremity and a predisposition to pulmonary embolism. Abnormal thrombus formation further characterizes disseminated intravascular coagulopathy commonly associated with both vascular systems during septic shock, certain viral infections and cancer, a condition wherein there is a rapid consumption of coagulation factors and systemic coagulation which results in the formation of life-threatening thrombi occurring throughout the microvasculature leading to wide-spread organ failure.

The following examples are offered by way of illustration and not by way of limitation. The disclosures of all citations in the specification are expressly incorporated herein by reference.

### EXAMPLES

### Example I

### Identification and Characterization of Peptides that Bind FVIIa and Inhibit FX Activation and Clotting

### Methods

*Phage Libraries -* The random sequence polyvalent peptide phage libraries have been described previously (Lowman, H. B., *et al*., *Biochemistry* 37:8870 (1998)). The peptide libraries were of the form X_{*i*}CX_{*j*}CX_{*k*} (where X was any of the 20 naturally ocurring L-amino acids and *j* ranged from 4-10 and *i* + *j* + *k* = 18), an unconstrained library X₂₀, and X₄CX₂GPX₄CX₄. Each of the 10 libraries has in excess of 10⁸ clones.

*Selection Conditions -* TF₁₋₂₄₃ (Paborsky, L. R., *et al*., *J. Biol*. *Chem*. 266: 21911 (1991)) or recombinant human FVIIa (2 µg/ml each) were immobilized directly to Maxisorp plates (Nunc) in 50 mM ammonium bicarbonate, pH 9.3 by incubating overnight at 4 °C. Wells were blocked using Sorting Buffer (50 mM HEPES, pH7.2, 5 mM CaCl₂, 5 mM MgCl₂, 150 mM NaCl, 1% BSA) for 1 h at 25 °C. Recombinant human FVIIa (2 µg/ml) in Sorting Buffer was added for 30 min to wells previously coated and blocked with TF to form the TF-FVIIa complex. Phage from the libraries described above were pooled into 3 groups. Pool A contained X_{*i*}CX_{*j*}CX_{*k*} where *j* = 5-7; Pool B contained X₄CX₂GPX₄CX₄, X₂₀ and X_{*i*}CX_{*j*}CX_{*k*} where *j* = 4; Pool E contained X_{*i*}CX_{*j*}CX_{*k*} where *j* = 8-10. Phage from each pool were incubated with the immobilized targets in Sorting Buffer for 3 h at 25 °C; generally about 5 x 10¹⁰ phage were added at the beginning of each round. Unbound phage were removed by repetitive washing with Wash Buffer (50 mM HEPES, pH7.2, 150 mM NaCl, 0.005% Tween 20); remaining phage were eluted with 500 mM KCl, 10 mM HCl, pH2. The eluted phage were then propagated in XL1-Blue cells with VCSM13 helper phage (Stratagene) overnight at 37 °C. Enrichment could be monitored by 'titering the number of phage which bound to a target coated well compare to a well coated with BSA.

*FX Activation Assay -* Activation of FX by TF-FVIIa was monitored at room temperature as a function of peptide concentration. Each assay sample contained 100 µl of 460 pM relipidated TF₁₋₂₄₃ (Kelley, R. F., *et al. Blood* 89:3219-3227 (1997)) and 30 pM FVIIa in HBS/Ca buffer (20 mM HEPES, pH 7.4, 5 mM CaCl₂, 150 mM NaCl, 0.1 % PEG 8000); after 20 min, 25 µl of peptide diluted in HBS/Ca Buffer was added. Following a 30 min incubation the reaction was initiated by the addition of 25 µl of 1 µM FX in HBS/Ca (Note: this yields a final concentration of 306 pM TF_{PC}, 20 pM FVIIa, and 166 nM FX). For kinetic analysis, the final concentration of FX was varied from between 20 and 500 nM. Aliquots of 25 µl were removed at 1, 3, 5, 7 and 9 min and quenched in 25 µl of 50 mM EDTA. The FXa generated in each aliquot could be measured by the addition of 100 µl of 250 µM Spectrozyme fXa (American Diagnostica), 50 mM Tris, pH 8, 50 mM NaCl, 0.0025 % Triton X-100. The rate of FXa generated at each peptide concentration was proportional to the initial slope of the absorbance at 405 nm vs. time. Sigmoidal curves were fit to a four-parameter equation by nonlinear regression analysis (Marquardt, *J. Soc. Indust. Appl. Math*. 11:431-441 (1963)); the concentration of each peptide required to give a half-maximal signal in the assay was calculated from the curves and is referred to as the IC₅₀ value.

*Clotting Assays -* Prothrombin time (PT) and activated partial thromboplastin time (APTT) clotting time assays were performed in citrated pooled normal plasmas (human or various animal species). Clotting times were determined using an ACL 300 Automated Coagulation Analyzer (Coulter Corp., Miami, Florida) and commercially available reagents as follows.

For the PT assay, aqueous solutions of inhibitor at various concentrations are added to citrated pooled normal plasma in a ratio of 1 part inhibitor to 9 parts plasma. Following a 30 min incubation, these mixtures are added to the sample cups of the ACL 300 Analyzer. Innovin® (Dade International Inc., Miami, Florida), a mixture of human relipidated tissue factor and Ca²⁺ ions is added to the reagent cup. Precise volumes of sample and Innovin® (50 µl sample, 100 µl Innovin®) are automatically transferred to cells of an acrylic rotor that is pre-equilibrated to 37 °C. Following a 2 min incubation period, coagulation is initiated when the two components are mixed together by centrifugation. Coagulation is monitored optically and clotting time is reported in seconds. In this system, the clotting time of control plasmas (plasma plus inhibitor diluent) is typically 8 to 10 seconds. The fold prolongation is the clotting time of the inhibitor relative to the clotting time of the control.

For the APTT assay, inhibitor and plasma are mixed together and transferred to the ACL 300 Analyzer sample cups as described above. Actin FS® and CaCl₂ (Dade International Inc., Miami, Florida), are added to reagent cups 1 and 2 respectively. Precise volumes of sample (53 µl) and Actin FS® (53 µl) are automatically transferred to cells of a rotor pre-equilibrated at 37 °C and mixed by centrifugation. Following a 2 min activation period, coagulation is initiated by the addition of CaCl₂ (53 µl). Coagulation is monitored optically and clotting time is reported in seconds. APTT of plasma controls is typically 12 to 32 seconds, depending upon the species of plasma used in the assay. The fold prolongation is the clotting time of the inhibitor relative to the clotting time of the control.

*Phage ELISA* - The ability of peptides to compete with peptide-phage for binding to TF-FVIIa was monitored using a phage ELISA. Dilutions of peptide in Sorting Buffer were added to microtiter plates coated with the TF-FVIIa complex (as described above) for 30 min. Approximately 10¹¹ monovalent phage displaying the TF100 peptide sequence were then added for an additional 15 min. The microtiter plate was washed with Wash Buffer and the phage bound to FVIIa were detected with an anti-gVIII/HRP monoclonal antibody conjugate (HRP/Anti-M13 Conjugate, Pharmacia Amersham Biotech). The amount of HRP bound was measured using ABTS/H₂O₂ substrate and monitoring the absorbance at 405 nm. The absorbance at 405 nm was plotted versus the concentration of peptide originally added to the well. Sigmoidal curves were fit to a four-parameter equation by nonlinear regression analysis (Marquardt, J., *Soc. Indust. Appl*. *Math*. 11:431-441 (1963); the concentration of each peptide required to give a half-maximal signal in the assay was calculated from the curves and is referred to as the IC₅₀ value.

*Partial and Complete Randomization on Monovalent Phage* - Monovalent libraries which display a single copy of a peptide on the surface of phage fused through a linker sequence to the tail protein coded for by gIII were constructed using single-stranded template-directed mutagenesis (Kunkel, T. A., *et al*., *Methods Enzymol*. 204:125-139 (1991)) of the phagemid t4.g3. Phagemid t4.g3 is a derivative of pA4G32 (Dennis, M. S., and Lazarus, R. A., *J. Biol*. *Chem*. 269:22129-22136 (1994)) where the coding sequence for APPI fused to gIII has been replaced by a 60 bp spacer fused in frame to a linker sequence and gIII; in addition, the CMP^{r} gene has been inserted into a unique hincII site in the AMP^{r} gene. The change in drug resistance was designed to eliminate contamination by related although weaker affinity polyvalent clones which could take over the population through avidity effects (Cwirla, S. A., *et al., Proc. Natl. Acad. Sci USA* 87:6378-6381 (1990)). Partially randomized libraries were designed to maintain a bias towards the peptide sequences identified from the initial polyvalent libraries.while allowing a 50% mutation rate at each amino acid position. This mutation rate was attained by synthesizing the oligos with a 70-10-10-10 mixture of bases (where each base in the doped region of the oligo is coupled using a mixture containing 70% of the base contributing to wild-type sequence and 10% each of the other 3 bases). In contrast, complete randomization in libraries was obtained by synthesizing oligos using NNS for particular codons in order to fully randomize portions of a displayed peptide while keeping other portions of the sequence constant.

*Peptide Synthesis -* Peptides were synthesized by either manual or automated (Perseptive Pioneer) Fmoc-based solid phase synthesis on a 0.25 mmol scale using a PEG-polystyrene resin (Bodansky, M., *Principles of Peptide Synthesis* (Springer, Berlin, 1984). Coupling of each amino acid was accomplished with 2-(H-Benzotriazole-1-yl)-1,1,3,3-tetramethyluronium hexafluorophospahte (HBTU) and N-Hydroxybenzotriazole (HOBt) with diisopropylethylamine (DIPEA) in dimethylacetamide (DMA). Peptides ending in a carboxyl-terminal amide were prepared on Rink amide resin. Acetylation of the amino terminus was accomplished with acetic anhydride in 10% triethylamine in dichloromethane. Side-chain protecting groups were removed and the peptide was cleaved from the resin with 95 % trifluoroacetic acid (TFA) and 5 % triisopropylsilane. A saturated iodine solution in acetic acid was added to oxidize the disulfide bonds. Peptides were purified by reversed-phase HPLC using a water/acetonitrile gradient containing 0.1 % TFA and lyophilized. Peptides were >95 % pure by analytical HPLC and their identity was verified by mass spectrometry.

*Production of peptide-Z fusions -* Phage peptides selected for binding to TF-FVIIa were expressed and secreted from *E. coli* (27C7) as fusions to the Z domain from protein A using a flexible linker (GGGSGG) (SEQ ID NO:99). Oligos were designed to insert the coding sequence for the phage-peptide sequences between the stII signal sequence and the Z domain in the plasmid pZCT (Starovasnik *et al*., *Protein Sci.* 8:1423-1431 (1999)). Cells were grown in phosphate limiting media and peptide-Z fusions were purified from the media using an IgG affinity column as described (Dennis *et al*., *Proteins: Structure*, *Function, and Genetics* 15:312-321 (1993)).

*Production of TF183 and TF183b-* TF183 was expressed in *E. coli* fused through a flexible linker to the Z domain of protein A as described above. The TF183 peptide, EEWEVLCWTWETCER (SEQ ID NO:23), could be isolated from the TF183-Z fusion by mild digestion with trypsin followed by reverse phase HPLC. Specific N-terminal labeling of purified TF183 with biotin to make TF183b was accomplished using NHS-LC-biotin (Pierce) according to the manufacturer's recommendations.

*FVIIa Binding ELISA* - The ability of peptides to compete with biotinylated TF183 (TF183b) (or other peptides described herein that could be biotinylated as described) for binding to FVIIa was monitored using a FVIIa Binding ELISA or a TF-FVIIa Binding ELISA. Microtiter plates were coated overnight with 2 µg/ml recombinant human FVIIa or 2 µg/ml TF₁₋₂₄₃ (Paborsky, L. R., *et al*., *J*. *Biol. Chem*. 266: 21911 (1991)) in 50 mM ammonium bicarbonate pH 9 at 4 °C; all other steps were performed at room temperature. Plates were then blocked with 1 % BSA in Assay Buffer (50 mM HEPES, pH 7.2, 5 mM CaCl₂, 150 mM NaCl). For the TF-FVIIa ELISA, recombinant human FVIIa (2 µg/ml) in 1% BSA in Assay Buffer was added for 30 min to the wells previously coated and blocked with TF to form the TF-FVIIa complex. Dilutions of peptide in Assay Buffer plus 0.05% Tween 20 were added to the microtiter plate along with 5 nM TF183b for 1 h. The microtiter plate was washed 3 times with Assay Buffer plus 0.05% Tween 20 and the biotinylated-peptide bound was detected with a Streptavidin/HRP conjugate (Streptavidin-POD, Roche Molecular Biochemicals). The amount of HRP bound was measured using ABTS/H₂O₂ substrate (Kirkegaard and Perry Laboratories) and monitoring the absorbance at 405 nm. The absorbance at 405 nm was plotted versus the concentration of peptide originally added to the well. Sigmoidal curves were fit to a four-parameter equation by nonlinear regression analysis (Marquardt, *J. Soc. Indust*. *Appl. Math*. 11:431-441 (1963); the concentration of each peptide required to give a half-maximal signal in the assay was calculated from the curves and is referred to as the IC₅₀ value.

*Screening Assay Using the FVIIa Binding ELISA -* The FVIIa Binding ELISA described above can also be used to screen for any compound that would block peptides of the present invention from binding to FVIIa. This could be carried out as described above or modified as described below. Thus, a competitive binding assay was established for use in high-throughput screening of chemical libraries for the purpose of identifying inhibitors of peptide binding. The assay is performed in opaque white, high-binding, 384-well plates coated with 1 µg/ml FVIIa and blocked with BSA. Sample, control, or assay buffer (20 µl) and biotinylated peptide (*e*.*g*., TF183b or other peptides described herein that could be biotinylated as described) (20 µl) are added to each well, and the plates are incubated for 1 h at room temperature. Sample or control may compete with the biotinylated peptide for binding to the FVIIa on the plate. The unbound biotinylated peptide is removed by washing the plate six times, and 40 µl of streptavidin-europium are added. During the subsequent 30 min incubation, the streptavidin-europium binds to the biotinylated peptide remaining on the plate. After washing six times to remove the unbound streptavidin-europium, 40 µl of enhancement solution are added to each well to dissociate the europium from the existing nonfluorescent chelate and to replace this with a highly fluorescent chelate. The fluorescence is read on a Wallac Victor microplate reader with excitation at 340 nm and emission at 615 nm following a 100 µsecond delay. The percent inhibition of binding is calculated relative to controls with assay buffer as sample.

### Results

*Polyvalent Peptide-Phage that bind to TF-FVIIa -* Polyvalent peptide libraries were sorted in 3 pools (designated A, B and E) against immobilized TF-FVIIa. Polyvalent phage display (Scott, J. K., and Smith, G. P., *Science* 249:386-390 (1990); Lowman, H. B., *Annu. Rev. Biophys. Biomol. Struct* 26:401-424 (1997); Wells, J. A., and Lowman, H. B., *Curr. Opin*. *Biotechnol*. 3:355-362 (1992)) was used to enhance binding through avidity effects. After four rounds of selection and amplification, the enrichment for Pool A, the number of phage eluted from a well coated with TF-FVIIa divided by the number of phage eluted from a well coated with BSA, was 10,000-fold. The DNA from six random clones in each pool were sequenced. The clones in Pool A were all siblings from a single clone with the deduced peptide sequence: SAEWEVLCWTWEGCGSVGLV (SEQ ID NO:1); designated TF53. Phage bearing this sequence bound specifically to immobilized FVIIa or TF-FVIIa but did not bind to wells coated with either TF or BSA. Additionally, this clone bound to both covalently and noncovalently active site blocked TF-FVIIa, where the active site of FVIIa was alkylated with biotinylated EGR chloromethylketone or blocked by TF7I-C, a Kunitz domain inhibitor (Dennis, M. S., and Lazarus, R. A., *J. Biol. Chem*. 269:22129-22136 (1994)), indicating that the peptide bound to FVIIa, but at a site distinct from the active site - an exosite.

*Partial Randomization* - The initial peptide libraries that were designed encoded a potential diversity of greater than 20²⁰ (10²⁶) different clones while the actual libraries that were made contained approximately only 10⁹ clones, a very small fraction of the potential diversity. In order to narrow the search and yet further explore the peptide diversity within the area of the initially selected peptides, a partial randomization technique was employed. This technique maintains a bias towards the wild-type sequence while introducing a 50% mutation rate (at the amino acid level) at each amino acid position; thus, on average, a phage displayed 20 amino acid peptide would acquire 10 random mutations. In addition, anticipation of further affinity improvements led us to construct these libraries on monovalent phage via fusion to gIII in order to eliminate avidity effects (Lowman, H. B., *Annu. Rev. Biophys. Biomol. Struct*. 26:401-424 (1997); Lowman, H. B., *et al*., *Biochemistry* 30:10832-10838 (1991)); Wells, J. A., and Lowman, H. B., *Curr. Opin*. *Biotechnol*. 3:355-362 (1992)).

A monovalent partial randomization library corresponding to the TF53 sequence was constructed and sorted for four rounds on TF-FVIIa. Enrichment of 100,000-fold was observed. Again, random clones were selected and sequenced; the deduced peptide sequences are shown in Table I below.

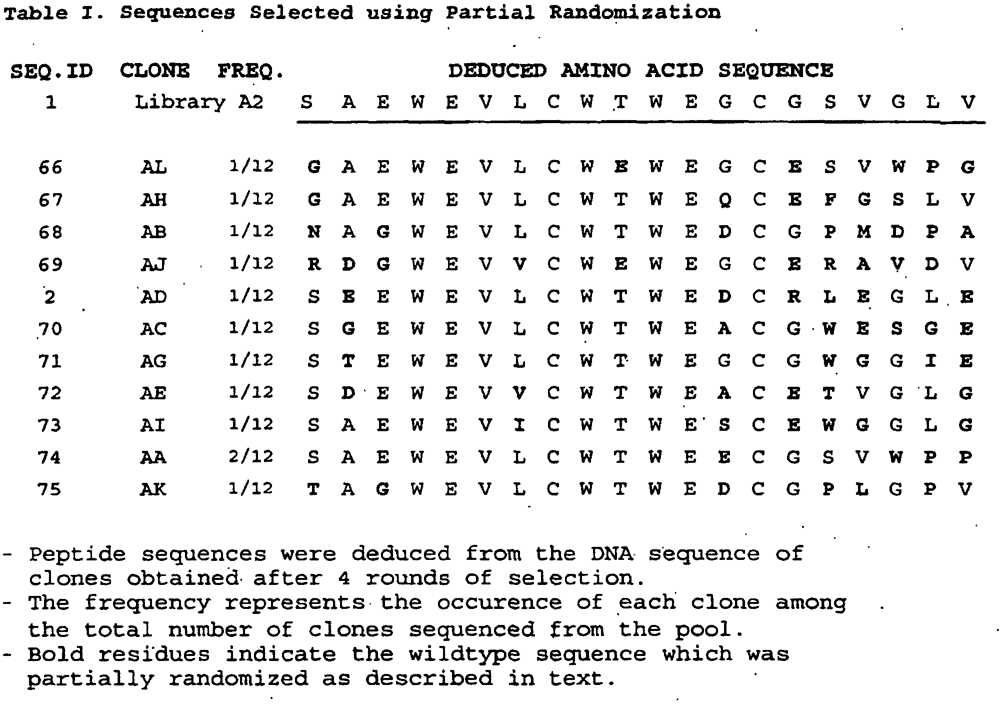

Several amino acid positions in the TF53 sequence were retained 100% yet multiple codons were observed at many of these positions; wild-type amino acids were still represented at each position reflecting the library design. Residues strongly retained following partial randomization may be crucial for binding either through direct contacts or for structural reasons. The large variation in selected sequences at the C terminal suggested this region maybe less important for binding to TF-FVIIa.

*Full maturation* - To complete the affinity maturation, a third set of libraries was constructed which fixed positions that were 100% conserved and fully randomized the remaining positions. In addition, the role of residues flanking the disulfide loop was addressed by constructing libraries with either portions of the amino and carboxyl terminal or both missing. Thus, three monovalent libraries were constructed and are described in Table II. Enrichment of 100,000-fold was observed by each library by round four; the sequences from random clones are presented in Table II. Even though these libraries (which fully randomized 12 amino acid positions) were far from complete, a comparison of the three libraries demonstrated a clear consensus for the optimum amino acid at most positions.

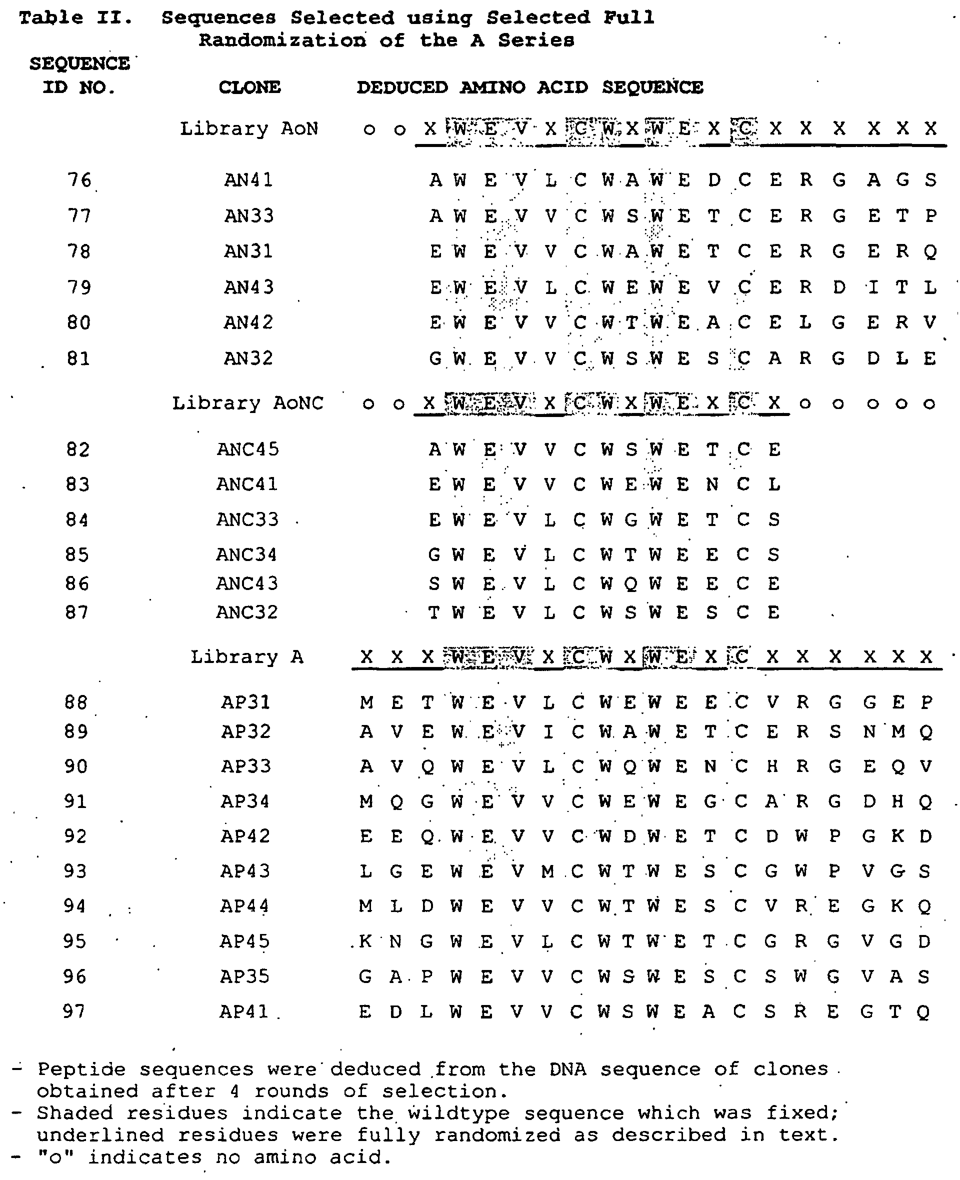

*Characterization of Peptides that bind to TF-FVIIa* - In order to assess the activity of the sequences selected from the phage displayed libraries, peptides corresponding to sequences selected from these libraries were chemically synthesized. Thus, peptide TF57 (SEEWEVLCWTWEDCRLEGLE) (SEQ ID NO:2), corresponding to a partially randomized-phage derived clone from library A2, and peptides TF100 (EEWEVLCWTWETCERGEG) (SEQ ID NO:18) and TF183 (EEWEVLCWTWETCER) (SEQ ID NO:23), corresponding to the consensus from the fully matured sequence, were chemically synthesized or purified from Z fusions expressed in *E. coli*. Data from these peptides as well as the TF100Z fusion are presented in the figures below.

Although peptides were selected only for binding to the TF-FVIIa complex, we were interested in finding functionally relevant peptides, *i.e.*, peptides that would inhibit the TF-FVIIa catalyzed activation of FX to FXa in a dose-dependent manner, by interfering with the binding and/or turnover of FX. Thus they were tested for their ability to inhibit FX activation; the inhibition of FX activation by selected peptides is shown in Fig. 1. The sequences of selected peptides and IC₅₀ values for inhibiting FX activation are shown in Figure 4. Many of the peptides were tested as peptide-Z fusions as indicated. Sequences derived later in the maturation process were more potent demonstrating the effectiveness of this procedure.

In agreement with their ability to block FX activation using purified components, the peptides described herein were potent anticoagulants. The inhibition of the TF-dependent extrinsic clotting pathway, as measured by the dose-dependent prolongation of the prothrombin time (PT), is shown in Fig. 2A. It is significant that we see no evidence for prolonging the clotting time in the surface-dependent intrinsic pathway, as determined by the activated partial thromboplastin time (APTT) (Fig. 2B). This implies that these peptides do not inhibit any of the serine proteases involved in the intrinsic pathway, which include thrombin, FXa, FIXa, FXIa, plasma kallikrein, and FXIIa.

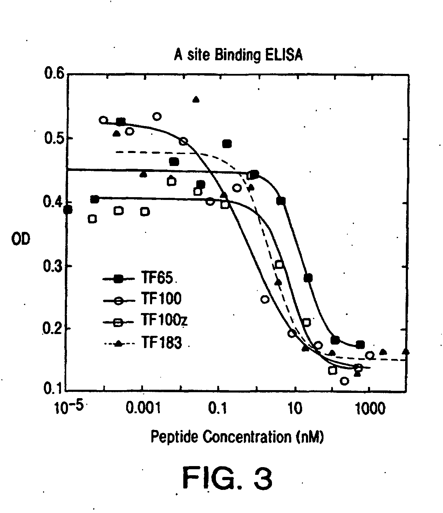

*FVIIa Binding ELISA* - The ability of peptides to compete with a biotinylated version of TF183 (TF183b) (or other peptides described herein that could be biotinylated as described) for binding to FVIIa was monitored using a FVIIa Binding ELISA; the inhibition of TF183b binding to FVIIa or TF-FVIIa by selected peptides is shown in Fig. 3. The sequences of selected peptides and the IC₅₀ values for their inhibition of the binding of TF183b to either FVIIa or TF-FVIIa is shown in Figure 4. The FVIIa Binding ELISA can also be used to screen for any compound that would block peptides of the present invention from binding to FVIIa. This could be carried out using a variety of reagents to detect the biotinylated peptide: Furthermore, a variety of peptides described herein could be used to develop the same type of assay. Thus, a competitive binding assay was established for use in high-throughput screening of chemical libraries for the purpose of identifying inhibitors of peptide binding.

## Claims

1. A peptide which:
i) comprises the sequence
ii) competes with SEQ ID NO: 4 for binding FVII/FVIIa in an *in vitro* assay and having between 1 and 8 amino acids of SEQ ID NO: 4 substituted according to the following:
Trp₁ is an amino acid selected from the group consisting of Trp, Phe, Tyr, Leu, Ile, Met, Val and Ala;
Glu₁ is any amino acid;
Val is an amino acid selected from the group consisting of Val, Trp, Phe, Tyr, Leu, Ile, Met and Ala;
Leu is an amino acid selected from the group consisting of Leu, Trp, Phe, Tyr, Ile, Met, Val and Ala;
Trp₂ is amino acid selected from the group consisting of Trp, Phe, Tyr, Leu, Ile, Met, Val and Ala;
Thr₁ is any amino acid;
Trp₃ is an amino acid selected from the group consisting of Trp, Phe, Tyr, Leu, Ile, Met, Val and Ala;
Glu₂ is any amino acid;
Thr₂ is any amino acid;
Glu₃ is any amino acid;
Arg is an amino acid selected from the group consisting of Arg, Lys, Leu, Trp, His, Met and Ile;
and
iii) comprises the peptide of ii).

2. The peptide of claim 1 which:
i) comprises the sequence
ii) competes with SEQ ID NO: 4 for binding FVII/FVIIa in an *in vitro* assay and having between 1 and 8 amino acids of SEQ ID NO: 4 substituted according to the following:
Trp₁ is an amino acid selected from the group consisting of Trp, Phe and Leu; Glu₁ is any amino acid;
Val is an amino acid selected from the group consisting of Val and Ile;
Leu is an amino acid selected from the group consisting of Leu, Ile, Met, Val and Ala;
Trp₂ is amino acid selected from the group consisting of Trp, Phe, Tyr; Leu and Met;
Thr₁ is any amino acid;
Trp₃ is an amino acid selected from the group consisting of Trp, Phe and Tyr;
Glu₂ is any amino acid;
Thr₂ is any amino acid;
Glu₃ is any amino acid;
Arg is an amino acid selected from the group consisting of Arg, Lys, Leu and Trp;
and
iii) comprises the peptide of ii).

3. The peptide of claim 2 having an IC₅₀ for FVII/FVIIa of less than 1 µM.

4. The peptide of claim 3 having an IC₅₀ for FVII/FVIIa of less than 100 nM.

5. The peptide of claim 4 having an IC₅₀ for FVII/FVIIa of less than 10 nM.

6. The peptide of claim 5 which binds FVII/FVIIa and inhibits FVIIa activity.

7. The peptide of claim 6 which blocks an activity associated with FVIIa selected from the group consisting of activation of FVII, activation of FIX and activation of FX.

8. The peptide of claim 7 which inhibits activation of FX.

9. The peptide of claim 8 having an IC₅₀ for inhibiting FX activation of less than 10 µM.

10. The peptide of claim 9 having an IC₅₀ for inhibiting FX activation of less than 100 nM.

11. The peptide of claim 10 having an IC₅₀ for inhibiting FX activation of less than 5 nM.

12. The peptide of claim 11 having the following formula:
Xᵢ-Cys₁-Xⱼ-Cys₂-Xₖ
wherein Xᵢ is absent or is between 1 and 100 amino acids; Xⱼ is 5 amino acids and Xₖ is absent or between 1 and 100 amino acids.

13. The peptide of claim 12 wherein Xᵢ and Xₖ are between 1 and 50 amino acids.

14. The peptide of claim 13 wherein Xᵢ and Xₖ are between 1 and 10 amino acids.

15. The peptide of claim 14 having the formula wherein Xaa₁ is an amino acid
Xaa₂ is an amino acid
Xaa₃ is an amino acid selected from the group consisting of Trp, Phe, Leu, Ala, Met and Val;
Xaa₄ is an amino acid;
Xaa₅ is an amino acid selected from the group consisting of Val, Ile, Ala, Trp and Tyr;
Xaa₆ is an amino acid selected from the group consisting of Leu, Ile, Met, Val and Ala;
Xaa₈ is selected from the group consisting of Trp, Phe, Leu, Met, Ala and Val; Xaa₉ is an amino acid
Xaa₁₀ is an amino acid selected from the group consisting of Trp, Phe, Met and Tyr;
Xaa₁₁ is an amino acid;
Xaa₁₂ is an amino acid;
Xaa₁₄ is an amino acid except proline;
Xaa₁₅ is an amino acid selected from the group consisting of Arg, Lys, Leu, Trp, His and Met;
Xaa₁₆ is an amino acid;
Xaa₁₇ is an amino acid; and
Xaa₁₈ is an amino acid.

16. The peptide of claim 15 wherein
Xaa₃ is selected from the group consisting of Trp, Phe, Leu and Ala;
Xaa₅ is selected from the group consisting of Val, Ile and Ala; and
Xaa₈ is selected from the group consisting of Trp, Phe, Leu, Met and Ala.

17. The peptide of claim 16 wherein
Xaa₃ is selected from the group consisting of Trp, Phe and Leu;
Xaa₅ is selected from the group consisting of Val and Ile;
Xaa₆ is selected from the group consisting of Leu, Ile, Met and Val;
Xaa₈ is selected from group consisting of Trp, Phe, Leu and Met;
Xaa₁₀ is selected from the group consisting of Trp and Phe; and
Xaa₁₅ is selected from the group consisting of Arg, Lys, Leu and Trp.

18. The peptide of claim 17 wherein -Xaa₈-Xaa₉-Xaa₁₀-Xaa₁₁-Xaa₁₂- is -Trp-Thr-Trp-Glu-Thr- (SEQ ID NO:100).

19. An in vitro method of inhibiting FVIIa activity comprising the step of:
a) contacting FVIIa with a peptide of claim 1 in the presence of tissue factor and under conditions which allow binding of the compound to FVIIa to occur.

20. A method for selecting a compound which blocks FVII/FVIIa activation of FX comprising the steps of:
(1) contacting FVII/FVIIa with a compound of claim 1 in the presence and absence of a candidate molecule under conditions which allow specific binding of the compound of claim 1 to FVII/FVIIa to occur;
(2) detecting the amount of specific binding of the compound of claim 1 to FVII/FVIIa that occurs in the presence and absence of the candidate compound wherein the amount of binding in the presence of the candidate compound relative to the amount of binding in the absence of the candidate molecule is indicative of the ability of the candidate compound to block FVII/FVIIa activation of FX.

21. An in vitro method of inhibiting the activation of FX comprising comprising contacting FVII/FVIIa with a compound that prevents the interaction of FVII/FVIIa with a compound of claim 1.

22. The method of inhibiting the activation of FX of claim 21 comprising contacting FVII/FVIIa with a compound that prevents the interaction of FVII/FVIIa with SEQ ID NO: 4.

23. A method of treating a TF/FVIIa mediated disease or disorder.

24. A peptide of any one of claims 1 to 18 for use in a method of medical treatment.

25. Use of the peptide of any one of claims 1 to 18 for the preparation of a medicament for treating a TF/FVIIa mediated disease or disorder in a host in need thereof comprising administering to the host a therapeutically effective amount of the peptide of claim 1.

26. The use of claim 25 wherein the TF/FVIIa mediated disease or disorder is tissue injury associated with blood coagulation.

27. A pharmaceutical composition comprising a compound of any one of claims 1 to 18 and a pharmaceutically acceptable carrier.

28. A pharmaceutical composition comprising the peptide of claim 27 and pharmaceutically acceptable carrier.

29. The composition of claim 28 which is suitable for inhalation.

30. The composition of claim 29 which is dry powder.

31. The composition of claim 29 which is a liquid.

## Patentansprüche

1. Peptid, das
i) die Sequenz umfasst,
ii) in einem In-vitro-Test mit Seq.-ID Nr. 4 um die Bindung von FVII/FVIIa konkurriert und von 1 bis 8 Aminosäuren von Seq.-ID Nr. 4 aufweist, die wie folgt substituiert sind:
Trp₁ ist eine Aminosäure, die aus der aus Trp, Phe, Tyr, Leu, Ile, Met, Val und Ala bestehenden Gruppe ausgewählt ist;
Glu₁ ist eine beliebige Aminosäure;
Val ist eine Aminosäure, die aus der aus Val, Trp, Phe, Tyr, Leu, Ile, Met und Ala bestehenden Gruppe ausgewählt ist;
Leu ist eine Aminosäure, die aus der aus Leu, Trp, Phe, Tyr, Ile, Met, Val und Ala bestehenden Gruppe ausgewählt ist;
Trp₂ ist eine Aminosäure, die aus der aus Trp, Phe, Tyr, Leu, Ile, Met, Val und Ala bestehenden Gruppe ausgewählt ist;
Thr₁ ist eine beliebige Aminosäure;
Trp₃ ist eine Aminosäure, die aus der aus Trp, Phe, Tyr, Leu, Ile, Met, Val und Ala bestehenden Gruppe ausgewählt ist;
Glu₂ ist eine beliebige Aminosäure;
Thr₂ ist eine beliebige Aminosäure;
Glu₃ ist eine beliebige Aminosäure;
Arg ist eine Aminosäure, die aus der aus Arg, Lys, Leu, Trp, His, Met und Ile bestehenden Gruppe ausgewählt ist; und
iii) das Peptid aus ii) umfasst.

2. Peptid nach Anspruch 1, das
i) die Sequenz umfasst,
ii) in einem In-vitro-Test mit Seq.-ID Nr. 4 um die Bindung von FVII/FVIIa konkurriert und von 1 bis 8 Aminosäuren von Seq.-ID Nr. 4 aufweist, die wie folgt substituiert sind:
Trp₁ ist eine Aminosäure, die aus der aus Trp, Phe und Leu bestehenden Gruppe ausgewählt ist;
Glu₁ ist eine beliebige Aminosäure;
Val ist eine Aminosäure, die aus der aus Val und Ile bestehenden Gruppe ausgewählt ist;
Leu ist eine Aminosäure, die aus der aus Leu, Ile, Met, Val und Ala bestehenden Gruppe ausgewählt ist;
Trp₂ ist eine Aminosäure, die aus der aus Trp, Phe, Tyr, Leu, und Met bestehenden Gruppe ausgewählt ist;
Thr₁ ist eine beliebige Aminosäure;
Trp₃ ist eine Aminosäure, die aus der aus Trp, Phe und Tyr bestehenden Gruppe ausgewählt ist;
Glu₂ ist eine beliebige Aminosäure;
Thr₂ ist eine beliebige Aminosäure;
Glu₃ ist eine beliebige Aminosäure;
Arg ist eine Aminosäure, die aus der aus Arg, Lys, Leu und Trp bestehenden Gruppe ausgewählt ist; und
iii) das Peptid aus ii) umfasst.

3. Peptid nach Anspruch 2 mit einem IC₅₀ für FVII/FVIIa von weniger als 1 µM.

4. Peptid nach Anspruch 3 mit einem IC₅₀ für FVII/FVIIa von weniger als 100 nM.

5. Peptid nach Anspruch 4 mit einem IC₅₀ für FVII/FVIIa von weniger als 10 nM.

6. Peptid nach Anspruch 5, das FVII/FVIIa bindet und FVIIa-Aktivität hemmt.

7. Peptid nach Anspruch 6, das eine mit FVIIa zusammenhängende Aktivität blockiert, das aus der aus Aktivierung von FVII, Aktivierung von FIX und Aktivierung von FX bestehenden Gruppe ausgewählt ist.

8. Peptid nach Anspruch 7, das die Aktivierung von FX hemmt.

9. Peptid nach Anspruch 8 mit einem IC₅₀ zur Hemmung von FX-Aktivierung von weniger als 10 µM.

10. Peptid nach Anspruch 9 mit einem IC₅₀ zur Hemmung von FX-Aktivierung von weniger als 100 nM.

11. Peptid nach Anspruch 10 mit einem IC₅₀ zur Hemmung von FX-Aktivierung von weniger als 5 nM.

12. Peptid nach Anspruch 11 mit der folgenden Formel:
Xᵢ-Cys₁-Xⱼ-Cys₂-Xₖ
worin Xᵢ nicht vorhanden ist oder 1 bis 100 Aminosäuren umfasst; Xⱼ 5 Aminosäuren umfasst und Xₖ nicht vorhanden ist oder 1 bis 100 Aminosäuren umfasst.

13. Peptid nach Anspruch 12, worin Xᵢ und Xₖ 1 bis 50 Aminosäuren umfassen.

14. Peptid nach Anspruch 13, worin Xᵢ und Xₖ 1 bis 10 Aminosäuren umfassen.

15. Peptid nach Anspruch 14 mit der Formel worin Xaa₁ eine Aminosäure ist;
Xaa₂ eine Aminosäure ist;
Xaa₃ eine Aminosäure ist, die aus der aus Trp, Phe, Leu, Ala, Met und Val bestehenden Gruppe ausgewählt ist;
Xaa₄ eine Aminosäure ist;
Xaa₅ eine Aminosäure ist, die aus der aus Val, Ile, Ala, Trp und Tyr bestehenden Gruppe ausgewählt ist;
Xaa₆ eine Aminosäure ist, die aus der aus Leu, Ile, Met, Val und Ala bestehenden Gruppe ausgewählt ist;
Xaa₈ eine Aminosäure ist, die aus der aus Trp, Phe, Leu, Met, Ala und Val bestehenden Gruppe ausgewählt ist;
Xaag eine Aminosäure ist;
Xaa₁₀ eine Aminosäure ist, die aus der aus Trp, Phe, Met und Tyr bestehenden Gruppe ausgewählt ist;
Xaa₁₁ eine Aminosäure ist;
Xaa₁₂ eine Aminosäure ist;
Xaa₁₄ eine Aminosäure mit Ausnahme von Prolin ist;
Xaa₁₅ eine Aminosäure ist, die aus der aus Arg, Lys, Leu, Trp, His und Met bestehenden Gruppe ausgewählt ist;
Xaa₁₆ eine Aminosäure ist;
Xaa₁₇ eine Aminosäure ist; und
Xaa₁₈ eine Aminosäure ist.

16. Peptid nach Anspruch 15, worin
Xaa₃ aus der aus Trp, Phe, Leu und Ala bestehenden Gruppe ausgewählt ist;
Xaa₅ aus der aus Val, Ile und Ala bestehenden Gruppe ausgewählt ist; und
Xaa₈ aus der aus Trp, Phe, Leu, Met und Ala bestehenden Gruppe ausgewählt ist.

17. Peptid nach Anspruch 16, worin
Xaa₃ aus der aus Trp, Phe und Leu bestehenden Gruppe ausgewählt ist;
Xaa₅ aus der aus Val und Ile bestehenden Gruppe ausgewählt ist;
Xaa₆ aus der aus Leu, Ile, Met und Val bestehenden Gruppe ausgewählt ist;
Xaa₈ aus der aus Trp, Phe, Leu und Met bestehenden Gruppe ausgewählt ist;
Xaa₁₀ aus der aus Trp und Phe bestehenden Gruppe ausgewählt ist; und
Xaa₁₅ aus der aus Arg, Lys, Leu und Trp bestehenden Gruppe ausgewählt ist.

18. Peptid nach Anspruch 17, worin -Xaa₈-Xaa₉-Xaa₁₀-Xaa₁₁-Xaa₁₂- -Trp-Thr-Trp-Glu-Thr (Seq.-ID Nr. 100) ist.

19. In-vitro-Verfahren zur Hemmung von FVIIa-Aktivität, folgenden Schritt umfassend:
a) Kontaktieren von FVIIa mit einem Peptid nach Anspruch 1 in Gegenwart eines Gewebefaktors und unter Bedingungen, welche die Bindung der Verbindung an FVIIa ermöglichen.

20. Verfahren zur Auswahl einer Verbindung, welche FVII/FVIIa-Aktivierung von FX blockiert, folgende Schritte umfassend:
(1) Kontaktieren von FVII/FVIIa mit einer Verbindung nach Anspruch 1 in Gegenwart und Abwesenheit eines Kandidatenmoleküls unter Bedingungen, welche die spezifische Bindung der Verbindung nach Anspruch 1 an FVII/FVIIa ermöglichen;
(2) Detektieren des Ausmaßes an spezifischer Bindung der Verbindung nach Anspruch 1 an FVII/FVIIa, die in Gegenwart und Abwesenheit der Kandidatenverbindung stattfindet, worin das Ausmaß der Bindung in Gegenwart der Kandidatenverbindung in Bezug auf das Ausmaß der Bindung in Abwesenheit des Kandidatenmoleküls ein Indikator für die Fähigkeit der Kandidatenverbindung ist, FVII/FVIIa-Aktivierung von FX zu blockieren.

21. In-vitro-Verfahren zur Hemmung der Aktivierung von FX, umfassend das Kontaktieren von FVII/FVIIa mit einer Verbindung, welche die Wechselwirkung von FVII/FVIIa mit einer Verbindung nach Anspruch 1 verhindert.

22. Verfahren zur Hemmung der Aktivierung von FX nach Anspruch 21, umfassend das Kontaktieren von FVII/FVIIa mit einer Verbindung, welche die Wechselwirkung von FVII/FVIIa mit Seq.-ID Nr. 4 verhindert.

23. Verfahren zur Behandlung einer durch TF/FVIIa vermittelten Krankheit oder Störung.

24. Peptid nach einem der Ansprüche 1 bis 18 zur Verwendung in einem medizinischen Behandlungsverfahren.

25. Verwendung eines Peptids nach einem der Ansprüche 1 bis 18 zur Herstellung eines Medikaments zur Behandlung einer durch TF/FVIIa vermittelten Krankheit in einem Wirt, der es benötigt, umfassend die Verabreichung einer therapeutisch wirksamen Menge des Peptids nach Anspruch 1 an den Wirten.

26. Verwendung nach Anspruch 25, worin die durch TF/FVIIa vermittelte Krankheit oder Störung Gewebeschädigung in Zusammenhang mit Blutgerinnung ist.

27. Pharmazeutische Zusammensetzung, umfassend eine Verbindung nach einem der Ansprüche 1 bis 18 und einen pharmazeutisch annehmbaren Träger.

28. Pharmazeutische Zusammensetzung, umfassend das Peptid nach Anspruch 27 und einen pharmazeutisch annehmbaren Träger.

29. Zusammensetzung nach Anspruch 28, die zum Inhalieren geeignet ist.

30. Zusammensetzung nach Anspruch 29, die ein Trockenpulver ist.

31. Zusammensetzung nach Anspruch 29, die eine Flüssigkeit ist.

## Revendications

1. Un peptide qui:
i) comprend la séquence
ii) entre en compétition avec ID SEQ NO: 4 pour relier FVII/FVIIa dans un essai *in vitro* et ayant entre 1 et 8 acides aminés de ID SEQ NO: 4 substitués conformément à ce qui suit:
Trp₁ est un acide aminé choisi dans le groupe se composant de Trp, Phe, Tyr, Leu, Ile, Met, Val et Ala;
Glu₁ est un acide aminé quelconque;
Val est un acide aminé choisi dans le groupe se composant de Val, Trp, Phe, Tyr, Leu, Ile, Met et Ala;
Leu est un acide aminé choisi dans le groupe se composant de Leu, Trp, Phe, Tyr, Ile, Met, Val et Ala;
Trp₂ est un acide aminé choisi dans le groupe se composant de Trp, Phe, Tyr, Leu, Ile, Met, Val et Ala;
Thr₁ est un acide aminé quelconque;
Trp₃ est un acide aminé choisi dans le groupe se composant de Trp, Phe, Tyr, Leu, Ile, Met, Val et Ala;
Glu₂ est un acide aminé quelconque;
Thr₂ est un acide aminé quelconque;
Glu₃ est un acide aminé quelconque;
Arg est un acide aminé choisi dans le groupe se composant de Arg, Lys, Leu, Trp, His, Met et Ile;
et
iii) comprend le peptide de ii).

2. Le peptide de la revendication 1 qui:
i) comprend la séquence
ii) entre en compétition avec ID SEQ NO: 4 pour relier FVII/FVIIa dans un essai *in vitro* et ayant entre 1 et 8 acides aminés de ID SEQ NO: 4 substitués conformément à ce qui suit:
Trp₁ est un acide aminé choisi dans le groupe se composant de Trp, Phe et Leu;
Glu₁ est un acide aminé quelconque;
Val est un acide aminé choisi dans le groupe se composant de Val et Ile;
Leu est un acide aminé choisi dans le groupe se composant de Leu, Ile, Met, Val et Ala;
Trp₂ est un acide aminé choisi dans le groupe se composant de Trp, Phe, Tyr, Leu et Met;
Thr₁ est un acide aminé quelconque;
Trp₃ est un acide aminé choisi dans le groupe se composant de Trp, Phe et Tyr;
Glu₂ est un acide aminé quelconque;
Thr₂ est un acide aminé quelconque;
Glu₃ est un acide aminé quelconque;
Arg est un acide aminé choisi dans le groupe se composant de Arg, Lys, Leu et Trp;
et
iii) comprend le peptide de ii).

3. Le peptide de la revendication 2 ayant une CI ₅₀ pour FVII/FVIIa inférieure à 1 µM.

4. Le peptide de la revendication 3 ayant une CI ₅₀ pour FVII/FVIIa inférieure à 100 nm.

5. Le peptide de la revendication 4 ayant une CI ₅₀ pour FVII/FVIIa inférieure à 10 nM.

6. Le peptide de la revendication 5 qui se lie à FVII/FVIIa et inhibe l'activité de FVIIa.

7. Le peptide de la revendication 6 qui b loque une activité associée à FVIIa choisie dans le groupe se composant de l'activation de FVII, l'activation de FIX et l'activation de FX.

8. Le peptide de la revendication 7 qui inhibe l'activation de FX.

9. Le peptide de la revendication 8 ayant une CI ₅₀ pour inhiber l'activation de FX inférieure à 10 µm.

10. Le peptide de la revendication 9 ayant une CI ₅₀ pour inhiber l'activation de FX inférieure à 100 nM.

11. Le peptide de la revendication 10 ayant une CI ₅₀ pour inhiber l'activation de FX inférieure à 5 nM.

12. Le peptide de la revendication 11 ayant la formule suivant:
Xᵢ-Cys₁-Xⱼ-Cys₂-Xₖ
dans laquelle X ᵢ est absent ou est entre 1 et 100 acides aminés; Xⱼ est 5 acides aminés et Xₖ est absent ou entre 1 et 100 acides aminés.

13. Le peptide de la revendication 12 dans leque 1 Xᵢ et Xₖ sont entre 1 et 50 acides aminés.

14. Le peptide de la revendication 13 dans lequel X ᵢ et Xₖ sont entre 1 et 10 acides aminés.

15. Le peptide de la revendication 14 ayant la formule dans laquelle Xaa₁ est un acide aminé
Xaa₂ est un acide aminé
Xaa₃ est un acide aminé choisi dans le groupe se composant de Trp, Phe, Leu, Ala, Met et Val;
Xaa₄ est un acide aminé;
Xaa₅ est un acide aminé choisi dans le groupe se composant de Val, Ile, Ala, Trp et Tyr;
Xaa₆ est un acide aminé choisi dans le groupe se composant de Leu, Ile, Met, Val et Ala;
Xaa₈ est choisi dans le groupe se composant de Trp, Phe, Leu, Met, Ala et Val;
Xaa₉ est un acide aminé
Xaa₁₀ est un acide amin é choisi dans le groupe se composant de Trp, Phe, Met est Tyr;
Xaa₁₁ est un acide aminé;
Xaa₁₂ est un acide aminé;
Xaa₁₄ est un acide aminé sauf la proline;
Xaa₁₅ est un acide aminé choisi dans le groupe se composant de Arg, Lys, Leu, Trp, His et Met;
Xaa₁₆ est un acide aminé;
Xaa₁₇ est un acide aminé; et
Xaa₁₈ est un acide aminé

16. Le peptide de la revendication 15 dans lequel
Xaa₃ est choisi dans le groupe se composant de Trp, Phe Leu, et Ala;
Xaa₅ est choisi dans le groupe se composant de Val, Ile et Ala; et
Xaa₈ est choisi dans le groupe se composant de Trp, Phe, Leu, Met et Ala.

17. Le peptide de la revendication 16 dans lequel
Xaa₃ est choisi dans le groupe se composant de Trp, Phe et Leu;
Xaa₅ est choisi dans le groupe se composant de Val et Ile;
Xaa₆ est choisi dans le groupe se composant de Leu, Ile, Met et Val;
Xaa₈ est choisi dans le groupe se composant de Trp, Phe, Leu et Met;
Xaa₁₀ est choisi dans le groupe se composant de Trp et Phe; et
Xaa₁₅ est choisi dans le groupe se composant de Arg, Lys, Leu et Trp.

18. Le peptide de la revendication 17 dans lequel

19. Un procédé in vitro d'inhibition de l'activité de FVIIa comprenant l'étape consistant à:
a) mettre en contact FVIIa avec un peptide de la revendication 1 en présence de thromboplastine tissulaire et sous des conditions qui permettent à la liaison du composé à FVIIa de se produire.

20. Un procédé de sélection d'un composé qui bloque l'activation FVII/FVIIa de FX comprenant les étapes consistant à:
(1) mettre en contact FVII/FVIIa avec un composé de la revendication 1, en présence et en l'absence d'une molécule candidate sous des conditions qui permettent de se produire la liaison spécifique du composé de la revendication 1 à FVII/FVIIa;
(2) détecter la quantité de liaison spécifique du composé de la revendication 1 à FVII/FVIIa qui se produit en présence et en l'absence du composé candidat dans lequel la quantité de liaison en présence du composé candidat par rapport à la quantité de liaison en l'absence de la molécule candidate est indicatrice de la capacité du composé candidat à bloquer l'activation FVII/FVIIa de FX.

21. Un procédé in vitro d'inhibition de l'activité de FX comprenant le fait de mettre en contact FVII/FVII a avec un composé qui empêche l'interaction de FVII/FVIIa avec un composé de la revendication 1.

22. Le procédé d'inhibition de l'activation de FX de la revendication 21 comprenant la mise en contact de FVII/FVIIa avec un composé qui empêche l'interaction de FVII/FVIIa avec ID SEQ NO: 4.

23. Un procédé de traitement d'une maladie ou d'un désordre à médiation TF/FVIIa.

24. Un peptide d'une quelconque des revendications 1 à 18 utilisable dans un procédé de traitement médical.

25. Utilisation du peptide d'une quelconque des revendications 1 à 18 pour la préparation d'un médicament pour traiter une maladie ou un désordre à médiation TF/FVIIa chez un hôte en ayant besoin comprenant le fait d'administrer à l'hôte une quantité thérapeutiquement efficace du peptide de la revendication 1.

26. L'utilisation de la revendication 25 dans laquelle la maladie ou le désordre à médiation TF/FVIIa est une blessure tissulaire associée à une coagulation du sang.

27. Une composition pharmaceutique comprenant un composé d'une quelconque des revendications 1 à 18 et un support pharmaceutiquement acceptable.

28. Une composition pharmaceutique comprenant le peptide de la revendication 27 et un support pharmaceutiquement acceptable.

29. La composition de la revendication 28 qui est appropriée pour inhalation.

30. La composition de la revendication 29 qui est une poudre sèche.

31. La composition de la revendication 29 qui est un liquide.


### Sequence Listing

<110> Genentech, Inc.
<120> FVIIa Antagonists
<130> P1639R1PCT
<150> US 60/147,627
<151> 1999-08-06
<150> US 60/150,315
<151> 1999-08-23
<160> 100
<210> 1
<211> 20
<212> PRT
<213> Artificial sequence
<220>
<223> synthetic peptide sequence
<400> 1
   Ser Ala Glu Trp Glu Val Leu Cys Trp Thr Trp Glu Gly Cys Gly
   1 5 10 15
   Ser Val Gly Leu Val
   20
<210> 2
<211> 20
<212> PRT
<213> Artificial sequence
<220>
<223> synthetic peptide sequence
<400> 2
   Ser Glu Glu Trp Glu Val Leu Cys Trp Thr Trp Glu Asp Cys Arg
   1 5 10 15
   Leu Glu Gly Leu Glu
   20
<210> 3
<211> 13
<212> PRT
<213> Artificial sequence
<220>
<223> synthetic peptide sequence
<400> 3
   Trp Glu Val Leu Cys Trp Thr Trp Glu Asp Cys Glu Arg
   1 5 10
<210> 4
<211> 13
<212> PRT
<213> Artificial sequence
<220>
<223> synthetic peptide sequence
<400> 4
   Trp Glu Val Leu Cys Trp Thr Trp Glu Thr Cys Glu Arg
   1 5 10
<210> 5
<211> 13
<212> PRT
<213> Artificial sequence
<220>
<223> synthetic peptide sequence
<400> 5
   Trp Glu Val Val Cys Trp Thr Trp Glu Thr Cys Glu Arg
   1 5 10
<210> 6
<211> 15
<212> PRT
<213> Artificial sequence
<220>
<223> synthetic peptide sequence
<400> 6
   Ser Glu Glu Trp Glu Val Leu Cys Trp Thr Trp Glu Asp Cys Arg
   1 5 10 15
<210> 7
<211> 14
<212> PRT
<213> Artificial sequence
<220>
<223> synthetic peptide sequence
<400> 7
   Glu Glu Trp Glu Val Leu Cys Trp Thr Trp Glu Asp Cys Arg
   1 5 10
<210> 8
<211> 13
<212> PRT
<213> Artificial sequence
<220>
<223> synthetic peptide sequence
<400> 8
   Glu Trp Glu Val Leu Cys Trp Thr Trp Glu Asp Cys Arg
    1               5                   10
<210> 9
<211> 12
<212> PRT
<213> Artificial sequence
<220>
<223> synthetic peptide sequence
<400> 9
   Trp Glu Val Leu Cys Trp Thr Trp Glu Asp Cys Arg
    1               5                   10
<210> 10
<211> 11
<212> PRT
<213> Artificial sequence
<220>
<223> synthetic peptide sequence
<400> 10
   Glu Val Leu Cys Trp Thr Trp Glu Asp Cys Arg
    1               5                   10
<210> 11
<211> 10
<212> PRT
<213> Artificial sequence
<220>
<223> synthetic peptide sequence
<400> 11
   Val Leu Cys Trp Thr Trp Glu Asp Cys Arg
   1               5                   10
<210> 12
<211> 8
<212> PRT
<213> Artificial sequence
<220>
<223> synthetic peptide sequence
<400> 12
   Cys Trp Thr Trp Glu Asp Cys Arg
   1               5
<210> 13
<211> 9
<212> PRT
<213> Artificial sequence
<220>
<223> synthetic peptide sequence
<400> 13
   Cys Trp Thr Trp Glu Asp Cys Glu Arg
   1               5
<210> 14
<211> 8
<212> PRT
<213> Artificial sequence
<220>
<223> synthetic peptide sequence
<400> 14
   Cys Trp Thr Trp Glu Asp Cys Glu
    1               5
<210> 15
<211> 9
<212> PRT
<213> Artificial sequence
<220>
<223> synthetic peptide sequence
<400> 15
   Cys Trp Thr Trp Glu Thr Cys Glu Arg
    1               5
<210> 16
<211> 8
<212> PRT
<213> Artificial sequence
<220>
<223> synthetic peptide sequence
<400> 16
   Cys Trp Thr Trp Glu Thr Cys Glu
    1               5
<210> 17
<211> 16
<212> PRT
<213> Artificial sequence
<220>
<223> synthetic peptide sequence
<400> 17
   Glu Trp Glu Val Leu Cys Trp Thr Trp Glu Thr Cys Glu Arg Gly
    1               5                  10                  15
   Glu
<210> 18
<211> 18
<212> PRT
<213> Artificial sequence
<220>
<223> synthetic peptide sequence
<400> 18
   Glu Glu Trp Glu Val Leu Cys Trp Thr Trp Glu Thr Cys Glu Arg
    1               5                   10                  15
   Gly Glu Gly
<210> 19
<211> 24
<212> PRT
<213> Artificial sequence
<220>
<223> synthetic peptide sequence
<400> 19
   Glu Glu Trp Glu Val Leu Cys Trp Thr Trp Glu Thr Cys Glu Arg
    1               5                   10                  15
   Gly Glu Gly Gly Gly Gly Ser Gly Gly
                   20
<210> 20
<211> 13
<212> PRT
<213> Artificial sequence
<220>
<223> synthetic peptide sequence
<400> 20
   Cys Trp Thr Trp Glu Thr Cys Glu Arg Gly Glu Gly Gln
    1               5                   10
<210> 21
<211> 16
<212> PRT
<213> Artificial sequence
<220>
<223> synthetic peptide sequence
<400> 21
   Glu Val Trp Glu Val Leu Cys Thr Asp Trp Glu Ser Cys Glu Trp
    1               5                   10                  15
   Gly
<210> 22
<211> 13
<212> PRT
<213> Artificial sequence
<220>
<223> synthetic peptide sequence
<400> 22
   Trp Glu Val Leu Cys Met Asp Trp Glu Thr Cys Glu Arg
    1               5                   10
<210> 23
<211> 15
<212> PRT
<213> Artificial sequence
<220>
<223> synthetic peptide sequence
<400> 23
   Glu Glu Trp Glu Val Leu Cys Trp Thr Trp Glu Thr Cys Glu Arg
    1               5                   10                  15
<210> 24
<211> 13
<212> PRT
<213> Artificial sequence
<220>
<223> synthetic peptide sequence
<400> 24
   Trp Lys Val Leu Cys Ala Thr Trp Ala Thr Cys Gln Arg
    1               5                  10
<210> 25
<211> 13
<212> PRT
<213> Artificial sequence
<220>
<223> synthetic peptide sequence
<400> 25
   Trp Glu Val Leu Cys Ala Thr Trp Glu Thr Cys Glu Arg
    1               5                  10
<210> 26
<211> 24
<212> PRT
<213> Artificial sequence
<220>
<223> synthetic peptide sequence
<400> 26
   Ala Glu Trp Glu Val Leu Cys Trp Thr Trp Glu Thr Cys Glu Arg
    1               5                  10                  15
   Gly Glu Gly Gly Gly Gly Ser Gly Gly
                   20
<210> 27
<211> 24
<212> PRT
<213> Artificial sequence
<220>
<223> synthetic peptide sequence
<400> 27
   Glu Ala Trp Glu Val Leu Cys Trp Thr Trp Glu Thr Cys Glu Arg
   1               5                   10                  15
   Gly Glu Gly Gly Gly Gly Ser Gly Gly
                   20
<210> 28
<211> 24
<212> PRT
<213> Artificial sequence
<220>
<223> synthetic peptide sequence
<400> 28
   Glu Glu Ala Glu Val Leu Cys Trp Thr Trp Glu Thr Cys Glu Arg
   1               5                   10                  15
   Gly Glu Gly Gly Gly Gly Ser Gly Gly
                   20
<210> 29
<211> 24
<212> PRT
<213> Artificial sequence
<220>
<223> synthetic peptide sequence
<400> 29
   Glu Glu Trp Ala Val Leu Cys Trp Thr Trp Glu Thr Cys Glu Arg
   1               5                   10                  15
   Gly Glu Gly Gly Gly Gly Ser Gly Gly
<210> 30
<211> 24
<212> PRT
<213> Artificial sequence
<220>
<223> synthetic peptide sequence
<400> 30
   Glu Glu Trp Glu Ala Leu Cys Trp Thr Trp Glu Thr Cys Glu Arg
   1               5                   10                  15
   Gly Glu Gly Gly Gly Gly Ser Gly Gly
                   20
<210> 31
<211> 24
<212> PRT
<213> Artificial sequence
<220>
<223> synthetic peptide sequence
<400> 31
   Glu Glu Trp Glu Val Ala Cys Trp Thr Trp Glu Thr Cys Glu Arg
   1               5                   10                  15
   Gly Glu Gly Gly Gly Gly Ser Gly Gly
                   20
<210> 32
<211> 24
<212> PRT
<213> Artificial sequence
<220>
<223> synthetic peptide sequence
<400> 32
   Glu Glu Trp Glu Val Leu Cys Ala Thr Trp Glu Thr Cys Glu Arg
   1               5                   10                  15
   Gly Glu Gly Gly Gly Gly Ser Gly Gly
                   20
<210> 33
<211> 24
<212> PRT
<213> Artificial sequence
<220>
<223> synthetic peptide sequence
<400> 33
   Glu Glu Trp Glu Val Leu Cys Trp Ala Trp Glu Thr Cys Glu Arg
   1               5                   10                  15
   Gly Glu Gly Gly Gly Gly Ser Gly Gly
                   20
<210> 34
<211> 24
<212> PRT
<213> Artificial sequence
<220>
<223> synthetic peptide sequence
<400> 34
   Glu Glu Trp Glu Val Leu Cys Trp Thr Ala Glu Thr Cys Glu Arg
   1               5                   10                  15
   Gly Glu Gly Gly Gly Gly Ser Gly Gly
                   20
<210> 35
<211> 24
<212> PRT
<213> Artificial sequence
<220>
<223> synthetic peptide sequence
<400> 35
   Glu Glu Trp Glu Val Leu Cys Trp Thr Trp Ala Thr Cys Glu Arg
   1               5                   10                  15
   Gly Glu Gly Gly Gly Gly Ser Gly Gly
                   20
<210> 36
<211> 24
<212> PRT
<213> Artificial sequence
<220>
<223> synthetic peptide sequence
.<400> 36
<210> 37
<211> 24
<212> PRT
<213> Artificial sequence
<220>
<223> Synthetic peptide sequence
<400> 37
   Glu Glu Trp Glu Val Leu Cys Trp Thr Trp Glu Thr Cys Ala Arg
   1               5                   10                  15
   Gly Glu Gly Gly Gly Gly Ser Gly Gly
                   20
<210> 38
<211> 24
<212> PRT
<213> Artificial sequence
<220>
<223> synthetic peptide sequence
<400> 38
   Glu Glu Trp Glu Val Leu Cys Trp Thr Trp Glu Thr Cys Glu Ala
   1 5 10 15
   Gly Glu Gly Gly Gly Gly Ser Gly Gly
   20
<210> 39
<211> 24
<212> PRT
<213> Artificial sequence
<220>
<223> synthetic peptide sequence
<400> 39
   Glu Glu Trp Glu Val Leu Cys Trp Thr Trp Glu Thr Cys Glu Arg
   1 5 10 15
   Ala Glu Gly Gly Gly Gly Ser Gly Gly
   20
<210> 40
<211> 24
<212> PRT
<213> Artificial sequence
<220>
<223> synthetic peptide sequence
<400> 40
   Glu Glu Trp Glu Val Leu Cys Trp Thr Trp Glu Thr Cys Glu Arg
   1 5 10 15
   Gly Ala Gly Gly Gly Gly Ser Gly Gly
   20
<210> 41
<211> 24
<212> PRT
<213> Artificial sequence
<220>
<223> synthetic peptide sequence
<400> 41
   Glu Glu Trp Glu Val Leu Cys Trp Thr Trp Glu Thr Cys Glu Arg
    1               5                  10                  15
   Gly Glu Ala Gly Gly Gly Ser Gly Gly
               20
<210> 42
<211> 24
<212> PRT
<213> Artificial sequence
<220>
<223> synthetic peptide sequence
<400> 42
   Glu Glu Trp Glu Ile Leu Cys Trp Thr Trp Glu Thr Cys Glu Arg
    1               5                  10                  15
   Gly Glu Gly Gly Gly Gly Ser Gly Gly
<210> 43
<211> 24
<212> PRT
<213> Artificial sequence
<220>
<223> synthetic peptide sequence
<400> 43
   Glu Glu Trp Glu Val Ile Cys Trp Thr Trp Glu Thr Cys Glu Arg
    1               5                  10                  15
   Gly Glu Gly Gly Gly Gly Ser Gly Gly
               20
<210> 44
<211> 24
<212> PRT
<213> Artificial sequence
<220>
<223> synthetic peptide sequence
<400> 44
   Glu Glu Trp Glu Val Met Cys Trp Thr Trp Glu Thr Cys Glu Arg
    1               5                  10                  15
   Gly Glu Gly Gly Gly Gly Ser Gly Gly
                   20
<210> 45
<211> 24
<212> PRT
<213> Artificial sequence
<220>
<223> synthetic peptide sequence
<400> 45
   Glu Glu Trp Glu Val Val Cys Trp Thr Trp Glu Thr Cys Glu Arg
    1               5                  10                  15
   Gly Glu Gly Gly Gly Gly Ser Gly Gly
                   20
<210> 46
<211> 24
<212> PRT
<213> Artificial sequence
<220>
<223> synthetic peptide sequence
<400> 46
   Glu Glu Trp Glu Val Leu Cys Phe Thr Trp Glu Thr Cys Glu Arg
    1               5                  10                  15
   Gly Glu Gly Gly Gly Gly Ser Gly Gly
                   20
<210> 47
<211> 24
<212> PRT
<213> Artificial sequence
<220>
<223> synthetic peptide sequence
<400> 47
   Glu Glu Trp Glu Val Leu Cys Leu Thr Trp Glu Thr Cys Glu Arg
    1               5                  10                  15
   Gly Glu Gly Gly Gly Gly Ser Gly Gly
                   20
<210> 48
<211> 24
<212> PRT
<213> Artificial sequence
<220>
<223> synthetic peptide sequence
<400> 48
   Glu Glu Trp Glu Val Leu Cys Met Thr Trp Glu Thr Cys Glu Arg
    1               5                  10                  15
   Gly Glu Gly Gly Gly Gly Ser Gly Gly
                   20
<210> 49
<211> 24
<212> PRT
<213> Artificial sequence
<220>
<223> synthetic peptide sequence
<400> 49
   Glu Glu Trp Glu Val Leu Cys Trp Thr Phe Glu Thr Cys Glu Arg
    1               5                  10                  15
   Gly Glu Gly Gly Gly Gly Ser Gly Gly
                   20
<210> 50
<211> 24
<212> PRT
<213> Artificial sequence
<220>
<223> synthetic peptide sequence
<400> 50
   Glu Glu Trp Glu Val Leu Cys Trp Thr Leu Glu Thr Cys Glu Arg
    1               5                  10                  15
   Gly Glu Gly Gly Gly Gly Ser Gly Gly
                   20
<210> 51
<211> 24
<212> PRT
<213> Artificial sequence
<220>
<223> synthetic peptide sequence
<400> 51
   Glu Glu Trp Glu Val Leu Cys Trp Thr Trp Arg Thr Cys Glu Arg
    1               5                  10                  15
   Gly Glu Gly Gly Gly Gly Ser Gly Gly
                   20
<210> 52
<211> 24
<212> PRT
<213> Artificial sequence
<220>
<223> synthetic peptide sequence
<400> 52
   Glu Glu Trp Glu Val Leu Cys Trp Thr Trp Gln Thr Cys Glu Arg
    1               5                  10                  15
   Gly Glu Gly Gly Gly Gly Ser Gly Gly
                   20
<210> 53
<211> 24
<212> PRT
<213> Artificial sequence
<220>
<223> synthetic peptide sequence
<400> 53
   Glu Glu Trp Glu Val Leu Cys Trp Thr Trp Glu Thr Cys Glu Lys
   1               5                   10                  15
   Gly Glu Gly Gly Gly Gly Ser Gly Gly
                   20
<210> 54
<211> 24
<212> PRT
<213> Artificial sequence
<220>
<223> synthetic peptide sequence
<400> 54
   Glu Glu Trp Glu Val Leu Cys Trp Thr Trp Glu Thr Cys Glu Leu
   1               5                   10                  15
   Gly Glu Gly Gly Gly Gly Ser Gly Gly
                   20
<210> 55
<211> 24
<212> PRT
<213> Artificial sequence
<220>
<223> synthetic peptide sequence
<400> 55
   Glu Glu Trp Glu Val Leu Cys Trp Thr Trp Glu Thr Cys Glu Trp
    1               5                  10                  15
   Gly Glu Gly Gly Gly Gly Ser Gly Gly
<210> 56
<211> 24
<212> PRT
<213> Artificial sequence
<220>
<223> synthetic peptide sequence
<400> 56
   Glu Glu Trp Glu Val Leu Ala Trp Thr Trp Glu Thr Ala Glu Arg
    1               5                  10                  15
   Gly Glu Gly Gly Gly Gly Ser Gly Gly
                   20
<210> 57
<211> 22
<212> PRT
<213> Artificial sequence
<220>
<223> synthetic peptide sequence
<400> 57
   Trp Glu Val Leu Cys Trp Thr Trp Glu Thr Cys Glu Arg Gly Glu
    1               5                  10                  15
   Gly Gly Gly Gly Ser Gly Gly
                   20
<210> 58
<211> 24
<212> PRT
<213> Artificial sequence
<220>
<223> synthetic peptide sequence
<400> 58
   Glu Glu Phe Glu Val Leu Cys Trp Thr Trp Glu Thr Cys Glu Arg
    1               5                  10                  15
   Gly Glu Gly Gly Gly Gly Ser Gly Gly
                   20
<210> 59
<211> 24
<212> PRT
<213> Artificial sequence
<220>
<223> synthetic peptide sequence
<400> 59
   Glu Glu Leu Glu Val Leu Cys Trp Thr Trp Glu Thr Cys Glu Arg
    1               5                  10                  15
   Gly Glu Gly Gly Gly Gly Ser Gly Gly
                   20
<210> 60
<211> 22
<212> PRT'
<213> Artificial sequence
<220>
<223> synthetic peptide sequence
<400> 60
   Phe Glu Val Leu Cys Trp Thr Trp Glu Thr Cys Glu Arg Gly Glu
    1               5                  10                  15
   Gly Gly Gly Gly Ser Gly Gly
                   20
<210> 61
<211> 22
<212> PRT
<213> Artificial sequence
<220>
<223> synthetic peptide sequence
<400> 61
   Phe Glu Val Leu Cys Met Thr Trp Glu Thr Cys Glu Arg Gly Glu
   1               5                   10                  15
   Gly Gly Gly Gly Ser Gly Gly
                   20
<210> 62
<211> 24
<212>. PRT
<213> Artificial sequence
<220>
<223> synthetic peptide sequence
<400> 62
   Glu Glu Tyr Glu Val Leu Cys Trp Thr Trp Glu Thr Cys Glu Arg
   1               5                   10                  15
   Gly Glu Gly Gly Gly Gly Ser Gly Gly
                   20
<210> 63
<211> 24
<212> PRT
<213> Artificial sequence
<220>
<223> synthetic peptide sequence
<400> 63
   Glu Glu Trp Glu Val Leu Cys Tyr Thr Trp Glu Thr Cys Glu Arg
   1               5                   10                  15
   Gly Glu Gly Gly Gly Gly Ser Gly Gly
                   20
<210> 64
<211> 24
<212> PRT
<213> Artificial sequence
<220>
<223> synthetic peptide sequence
<400> 64
   Glu Glu Trp Glu Val Leu Cys Trp Thr Tyr Glu Thr Cys Glu Arg
    1               5                  10                  15
   Gly Glu Gly Gly Gly Gly Ser Gly Gly
                   20
<210> 65
<211> 24
<212> PRT
<213> Artificial sequence
<220>
<223> synthetic peptide sequence
<400> 65
   Glu Glu Trp Glu Val Leu Cys Trp Thr Trp Glu Thr Cys Glu Trp
    1               5                  10                  15
   Lys Glu Gly Gly Gly Gly Ser Gly Gly
                   20
<210> 66
<211> 20
<212> PRT
<213> Artificial sequence
<220>
<223> synthetic peptide sequence
<400> 66
   Gly Ala Glu Trp Glu Val Leu Cys Trp Glu Trp Glu Gly Cys Glu
    1               5                  10                  15
   Ser Val Trp Pro Gly
                   20
<210> 67
<211> 20 .
<212> PRT
<213> Artificial sequence
<220>
<223> synthetic peptide sequence
<400> 67
   Gly Ala Glu Trp Glu Val Leu Cys Trp Thr Trp Glu Gln Cys Glu
   1               5                   10                  15
   Phe Gly Ser Leu Val
                   20
<210> 68
<211> 20
<212> PRT
<213> Artificial sequence
<220>
<223> synthetic peptide sequence
<400> 68
   Asn Ala Gly Trp Glu Val Leu Cys Trp Thr Trp Glu Asp Cys Gly
   1               5                   10                  15
   Pro Met Asp Pro Ala
<210> 69
<211> 20
<212> PRT
<213> Artificial sequence
<220>
<223> synthetic peptide sequence
<400> 69
   Arg Asp Gly Trp Glu Val Val Cys Trp Glu Trp Glu Gly Cys Glu
   1               5                   10                  15
   Arg Ala Val Asp Val
                   20
<210> 70
<211> 20
<212> PRT
<213> Artificial sequence
<220>
<223> synthetic peptide sequence
<400> 70
   Ser Gly Glu Trp Glu Val Leu Cys Trp Thr Trp Glu Ala Cys Gly
   1               5                   10                  15
   Trp Glu Ser Gly Glu
                   20
<210> 71
<211> 20
<212> PRT
<213> Artificial sequence
<220>
<223> synthetic peptide sequence
<400> 71
   Ser Thr Glu Trp Glu Val Leu Cys Trp Thr Trp Glu Gly Cys Gly
   1               5                   10                  15
   Trp Gly Gly Ile Glu
                   20
<210> 72
<211> 20
<212> PRT
<213> Artificial sequence
<220>
<223> synthetic peptide sequence
<400> 72
   Ser Asp Glu Trp Glu Val Val Cys Trp Thr Trp Glu Ala Cys Glu
   1               5                   10                  15
   Thr Val Gly Leu Gly
                   20
<210> 73
<211> 20
<212> PRT
<213> Artificial sequence
<220>
<223> synthetic peptide sequence
<400> 73
   Ser Ala Glu Trp Glu Val Ile Cys Trp Thr Trp Glu Ser Cys Glu
   1               5                   10                  15
   Trp Gly Gly Leu Gly
                   20
<210> 74
<211> 20
<212> PRT
<213> Artificial sequence
<220>
<223> synthetic peptide sequence
<400> 74
   Ser Ala Glu Trp Glu Val Leu Cys Trp Thr Trp Glu Glu Cys Gly
   1               5                   10                  15
   Ser Val Trp Pro Pro
                   20
<210> 75
<211> 20
<212> PRT
.<213> Artificial sequence
<220>
<223> synthetic peptide sequence
<400> 75
   Thr Ala Gly Trp Glu Val Leu Cys Trp Thr Trp Glu Asp Cys Gly
   1 5 10 15
   Pro Leu Gly Pro Val
   20
<210> 76
<211> 18
<212> PRT
<213> Artificial sequence
<220>
<223> synthetic peptide sequence
<400> 76
   Ala Trp Glu Val Leu Cys Trp Ala Trp Glu Asp Cys Glu Arg Gly
   1 5 10 15
   Ala Gly Ser
<210> 77
<211> 18
<212> PRT
<213> Artificial sequence
<220>
<223> synthetic peptide sequence
<400> 77
   Ala Trp Glu Val Val Cys Trp Ser Trp Glu Thr Cys Glu Arg Gly
   1 5 10 15
   Glu Thr Pro
<210> 78
<211> 18
<212> PRT
<213> Artificial sequence
<220>
<223> synthetic peptide sequence
<400> 78
   Glu Trp Glu Val Val Cys Trp Ala Trp Glu Thr Cys Glu Arg Gly
    1               5                   10                  15
   Glu Arg Gln
<210> 79
<211> 18
<212> PRT
<213> Artificial sequence
<220>
<223> synthetic peptide sequence
<400> 79
   Glu Trp Glu Val Leu Cys Trp Glu Trp Glu Val Cys Glu Arg Asp
    1               5                   10                  15
   Ile Thr Leu
<210> 80
<211> 18
<212> PRT
<213> Artificial sequence
<220>
<223> synthetic peptide sequence
<400> 80
   Glu Trp Glu Val Val Cys Trp Thr Trp Glu Ala Cys Glu Leu Gly
    1               5                   10                  15
   Glu Arg Val
<210> 81
<211> 18
<212> PRT
<213> Artificial sequence
<220>
<223> synthetic peptide sequence
<400> 81
   Gly Trp Glu Val Val Cys Trp Ser Trp Glu Ser Cys Ala Arg Gly
    1               5                  10                  15
   Asp Leu Glu
<210> 82
<211> 13
<212> PRT
<213> Artificial sequence
<220>
<223> synthetic peptide sequence
<400> 82
   Ala Trp Glu Val Val Cys Trp Ser Trp Glu Thr Cys Glu
    1               5                  10
<210> 83
<211> 13
<212> PRT
<213> Artificial sequence
<220>
<223> synthetic peptide sequence
<400> 83
   Glu Trp Glu Val Val Cys Trp Glu Trp Glu Asn Cys Leu
    1               5                  10
<210> 84
<211> 13
<212> PRT
<213> Artificial sequence
<220>
<223> synthetic peptide sequence
<400> 84
   Glu Trp Glu Val Leu Cys Trp Gly Trp Glu Thr Cys Ser
   1 5 10
<210> 85
<211> 13
<212> PRT
<213> Artificial sequence
<220>
<223> synthetic peptide sequence
<400> 85
   Gly Trp Glu Val Leu Cys Trp Thr Trp Glu Glu Cys Ser
   1 5 10
<210> 86
<211> 13
<212> PRT
<213> Artificial sequence
<220>
<223> synthetic peptide sequence
<400> 86
   Ser Trp Glu Val Leu Cys Trp Gln Trp Glu Glu Cys Glu
   1 5 10
<210> 87
<211> 13
<212> PRT
<213> Artificial sequence
<220>
<223> synthetic peptide sequence
<400> 87
   Thr Trp Glu Val Leu Cys Trp Ser Trp Glu Ser Cys Glu
   1 5 10
<210> 88
<211> 20
<212> PRT
<213> Artificial sequence
<220>
<223> synthetic peptide sequence
<400> 88
   Met Glu Thr Trp Glu Val Leu Cys Trp Glu Trp Glu Glu Cys Val
   1 5 10 15
   Arg Gly Gly Glu Pro
   20
<210> 89
<211> 20
<212> PRT
<213> Artificial sequence
<220>
<223> synthetic peptide sequence
<400> 89
   Ala Val Glu Trp Glu Val Ile Cys Trp Ala Trp Glu Thr Cys Glu
   1 5 10 15
   Arg Ser Asn Met Gln
   20
<210> 90
<211> 20
<212> PRT
<213> Artificial sequence
<220>
<223> synthetic peptide sequence
<400> 90
   Ala Val Gln Trp Glu Val Leu Cys Trp Gln Trp Glu Asn Cys His
   1 5 10 15
   Arg Gly Glu Gln Val
   20
<210> 91
<211> 20
<212> PRT
<213> Artificial sequence
<220>
<223> synthetic peptide sequence
<400> 91
   Met Gln Gly Trp Glu Val Val Cys Trp Glu Trp Glu Gly Cys Ala
   1 5 10 15
   Arg Gly Asp His Gln
<210> 92
<211> 20
<212> PRT
<213> Artificial sequence
<220>
<223> synthetic peptide sequence
<400> 92
   Glu Glu Gln Trp Glu Val Val Cys Trp Asp Trp Glu Thr Cys Asp
   1 5 10 15
   Trp Pro Gly Lys Asp
   20
<210> 93
<211> 20
<212> PRT
<213> Artificial sequence
<220>
<223> synthetic peptide sequence
<400> 93
   Leu Gly Glu Trp Glu Val Met Cys Trp Thr Trp Glu Ser Cys Gly
   1               5                   10                  15
   Trp Pro Val Gly Ser
                   20
<210> 94
<211> 20
<212> PRT
<213> Artificial sequence
<220>
<223> synthetic peptide sequence
<400> 94
   Met Leu Asp Trp Glu Val Val Cys Trp Thr Trp Glu Ser Cys Val
   1               5                   10                  15
   Arg Glu Gly Lys Gln
                   20
<210> 95
<211> 20
<212> PRT
<213> Artificial sequence
<220>
<223> synthetic peptide sequence
<400> 95
   Lys Asn Gly Trp Glu Val Leu Cys Trp Thr Trp Glu Thr Cys Gly
   1               5                   10                  15
   Arg Gly Val Gly Asp
                   20
<210> 96
<211> 20
<212> PRT
<213> Artificial sequence
<220>
<223> synthetic peptide sequence
<400> 96
   Gly Ala Pro Trp Glu Val Val Cys Trp Ser Trp Glu Ser Cys Ser
    1               5                  10                  15
   Trp Gly Val Ala Ser
                   20
<210> 97
<211> 20
<212> PRT
<213> Artificial sequence
<220>
<223> synthetic peptide sequence
<400> 97
   Glu Asp Leu Trp Glu Val Val Cys Trp Ser Trp Glu Ala Cys Ser
    1               5                  10                  15
   Arg Glu Gly Thr Gln
                   20
<210> 98
<211> 68
<212> PRT
<213> Staphylococcus aureus
<400> 98
   Ala Gln His Asp Glu Ala Val Asp Asn Lys Phe Asn Lys Glu Gln
    1               5                  10                  15
   Gln Asn Ala Phe Tyr Glu Ile Leu His Leu Pro Asn Leu Asn Glu
                   20                  25                  30
   Glu Gln Arg Asn Ala Phe Ile Gln Ser Leu Lys Asp Asp Pro Ser
                   35                  40                  45
   Gln Ser Ala Asn Leu Leu Ala Glu Ala Lys Lys Leu Asn Asp Ala
                   50                  55                  60
   Gln Ala Pro Asn Val Asp Met Asn
                   65
<210> 99
<211> 6
<212> PRT
<213> Artificial sequence
<220>
<223> peptide linker
<400> 99
   Gly Gly Gly Ser Gly Gly
    1               5
<210> 100
<211> 5
<212> PRT
<213> Artificial sequence
<220>
<223> synthetic peptide sequence
<400> 100
   Trp Thr Trp Glu Thr
    1               5